# EUROPEAN PATENT SPECIFICATION

(11) **EP 0 403 156 B1**
(45) Date of publication and mention of the grant of the patent: **10.09.1997**
(21) Application number: 90306178.6
(22) Date of filing: 07.06.1990
(51) Int. Cl.: C07K 16/46, C12P 21/08, C12N 15/62, A61K 39/395, C12N 5/20, A61K 47/48, C12N 5/10

(54) **Improved monoclonal antibodies against the human alpha/beta t-cell receptor, their production and use**
Monoklonale Antikörper gegen den humanen alpha/beta-T-Zellenrezeptor, ihre Herstellung und Verwendung
Anticorps monoclonaux améliorés contre le récepteur alpha/bêta des cellules T humaines, leur production et leur utilisation

(30) Priority: 07.06.1989 US 362549; 25.05.1990 US 529979
(43) Date of publication of application: 19.12.1990
(73) Proprietor: GENZYME CORPORATION, Framingham, Massachusetts 01701 (US); BEHRINGWERKE Aktiengesellschaft, 35001 Marburg (DE)
(72) Inventor: Kurrle, Roland, D-3550 Marburg (DE); Shearman, Clyde W., Bellingham, Massachussetts 02019 (US); Moore, Gordon P., Lexington, Massachusetts 02173 (US); Seiler, Fritz, D-3550 Marburg (DE)
(74) Representative: Sheard, Andrew Gregory

(56) References cited:
- WO-A-89/01783
- TRANSPLANTATION PROCEEDINGS vol. XX, no. 2(2), 1988, New York, USA pages 103 - 109; H. SCHLITT et al.: "Evidence for a cyclosporine-resistant pathway of T cell activation triggered via the CD3/T cell receptor complex"

## Description

The instant application discloses useful monoclonal antibodies (mAbs) against an epitope within the constant region of the human alpha/beta T-cell receptor (TCR), their production and use for immunosuppressive therapy in organ and bone marrow transplantation, in treatment of autoimmune diseases and for therapeutic applications in immunoregulation.

For decades Anti-leukocyte-antibodies for clinical and experimental use have been generated by immunizing, e.g. horses, rabbits, goats and rats with leukocytes, lymphocytes or subpopulations thereof or with various tumor cell lines. Specificity of such anti-leukocyte-globulin/Antithymocyte-globulin (ALG/ATG) preparations was usually obtained by careful selection of antigen sources or by absorption of undesired antibodies with different cell types such as erythrocytes, B-cells, selected cell lines, etc. This strategy, which resulted in high quality ALG/ATGs, requires a considerable expenditure of laboratory force, quality control, and the necessity to ascertain reproducible specificity from batch-to-batch. Within certain limitations, those ALG/ATGs made it possible to study leukocyte differentiation, the cellular origin of leukemia and lymphoma, to define T-cell subpopulations, and even to prepare antiidiotypic antisera.

The therapeutic efficacy of ALG/ATGs is well known, especially for immunosuppression in organ transplantation. In addition, ALG/ATGs have been used successfully to treat patients with aplastic anemia and for "purging" bone marrow cells within the context of bone marrow transplantation. Despite the success with ALG/ATG preparations it is accepted that even with a reasonable amount of laboratory effort, these polyclonal anti-leukocyte-antibodies might vary from batch-to-batch and the specificity of these antisera is limited.

Once the technique to produce mAbs was developed by Kohler and Milstein (Nature 225(1974)), it was possible to generate antibodies with much higher specificity as compared to ALG/ATG preparations. Since mAbs recognize not only distinct antigens, e.g. on cell surfaces, but also particular epitopes within such an antigen, they can be used with high efficacy to discriminate even between very similar cell populations, and to characterize the biochemical and functional aspects of the recognized antigen.

One use of mAbs is in targeting carcinomas so that they can be treated or diagnosed. This is disclosed in WO89/01783, for example, where a humanised antibody molecule having specificity for the TAG-72 antigen is disclosed. This molecule comprises CDRs of its variable domain which are derived from the-mouse monoclonal antibody B72.3. The remaining immunoglobulin parts of the molecule are derived from a human immunoglobulin.

mAbs have been most frequently and successfully used for immunosuppressive therapy in clinical organ transplantation. However, most mAbs have a broad immunosuppressive capacity, thus undesirably influencing functions of a wide spectrum of immune cells presumably not all involved in graft rejection.

Among others, the monoclonal antibody OKT3, directed against mature human T cells, has been extensively used for the treatment of patients undergoing acute allograft rejection after kidney transplantation (Russell P.S., Colvin R.B., Cosimi, A.B.: Annu. Rev. Med. 35:63, (1984) and Cosimi A.B., Burton R.C., Colvin, R.B. et al: Transplantation 32:535, (1981)). Moreover, OKT3 and rabbit complement were used for purging mature T-cells from donor marrow to prevent acute graft-v-host disease (GVHD) in allogeneic bone marrow transplantation (Prentice, H.G., Blacklock, H.A., Janossy, G. et al: Lancet 1:700, (1982) and Blacklock, H.A., Prentice, H.G., Gilmore, M.J. et al.:Exp. Hematol. 11:37, (1983)). Whereas OKT3 treatment seems to be effective in the prevention of GVHD in allogeneic bone marrow transplantation for acute leukemia, a combined in vitro/in vivo treatment with OKT3 failed to prevent GVHD during therapy for severe combined immunodeficiency (Hayward, A.R. et al.: Clin. Lab. Observ. 100:665, (1982)).

Treatment of T-cells with OKT3 elicits several responses inconsistent with immune suppression including T-cell activation, production of immune mediators and T3-modulation. The T3-antigen complex recognized by CD3-mAbs (e.g. OKT3) is postulated to play a crucial role during T-cell activation. Functional studies indicate that the T3-antigen complex is involved in specific immune functions as molecules functionally and physically associated with the respective T-cell receptor. Under physiological conditions the mere binding of OKT3 to T-cells results in T-cell activation. When T-cells are activated in the presence of accessory cells, OKT3 is highly mitogenic for T-cells from all donors, whereas for anti-T3 mAbs of the IgG1 isotype, nonresponsiveness caused by polymorphism in the accessory cell function has been described. Additionally, it has been reported that stimulation of human peripheral blood lymphocytes with OKT3 induces the production of immune mediators such as interferon (alpha-IFN) and interleukin-2 (IL-2) (Pang, R.H., Yip, Y.K., Vilcek, J.: Cell Immunol. 64:304, (1981) and Welte, K., Platzer E. Wang, C.Y., et al.: J. Immunol. 131:2356, (1983)). One of the earliest events after the binding of OKT3 to the T-cell membrane is the modulation of the T3 complex. T3 modulation occurs under appropriate conditions in vitro as well as in vivo and this mechanism, among others, seems to be responsible for the "escape" of T-cells during in vivo therapy with OKT3. Also antigenic modulation seems to play a critical role in T-cell activation.

In light of undesired effects of OKT3 described above, there was a need to create new mAbs having a specificity against mature lymphocytes and suitable for clinical application. Still another aspect and goal involves the coupling of such mAbs to cytotoxic agents (radioisotopes, toxins, enzymes, etc.) in order to improve their effectiveness in mediating cytolysis.

Another goal involved the modification, via genetic manipulation, of the mAb in order to produce chimeric antibodies having mixed murine and human characteristics in order to improve their effectiveness and/or lower their immunogenicity in patients.

Chimeric antibodies offer an additional advantage over murine mAb with regards to immunogenicity in patients. A chimeric antibody would retain the affinity and specificity of the parental murine mAb and eliminate the patient immune response to the murine constant regions. A further refinement involves humanization of the variable regions. Only the complementarity determing regions and selected framework amino acids necessary for antigen binding are maintained murine. The remaining framework regions are converted to human sequences. The resulting mAb of the present invention is thus essentially a human antibody with a much lower immunogenicity in patients.

According to the present invention there is provided a chimaeric antibody specific for an epitope on the human alpha/beta T-cell receptor (TCR) which is recognised by mAb BMA 031, the antibody comprising:
(a) a heavy chain whose variable region comprises the sequence of residues 1 to 120 of CIV-3 as shown in Table 6A; and
(b) a light chain whose variable region comprises the sequence of residues 1 to 107 of CIV-3 as shown in Table 6B. We have made a useful mAb secreted by the hybridoma cell line designated as BMA 031 by immunizing mice with human peripheral blood T-lymphocytes separated by the E-rosette-technique (so called E⁺-cells). The mAb secreted by BMA 031 (in the following likewise designated as BMA 031) is a murine monoclonal antibody of the IgG2b isotype with a specificity for the alpha- and beta-chain of the TCR/CD3 receptor complex. As compared to OKT3 or BMA 030 (both clustered as CD3-antibodies), GMA 031 only very weakly induces mediator release after binding to T-cells. It is highly effective in clinical application, e.g. kidney transplant for patients with increased immunological risk when given post or at the time of the transplant-operation. Since no major side effects were observed with GMA 031 even at doses of up to 50 mg/dose, it may be advantageously given at time of surgery (preferably via a first injection of 50 mg intravenously) followed by a second injection 48 hours after transplantation. Graft function was perfect in almost all cases.

BMA 031 defines, therefore, a valuable epitope on the alpha-beta TCR distinct from the epitope on CD3 recognized by OKT3 or by other mAbs against mature T-lymphocytes. In order to completely characterize this mAb and also to permit, by recombinant DNA techniques, exchange of the variable region frameworks outside of the hypervariable regions and exchange of the human constant region in place of the endogenous murine constant region, the DNA coding for the heavy and light chains of BMA 031 was cloned and sequenced.

The present inventors have recognised and defined the epitope on alpha/beta TCR. The present invention is directed to chimaeric antibodies directed against this epitope, having the characteristics set out in claim 1.

Preferred embodiments of the chimaeric antibodies of the present invention are described in claims 2 to 5.

A further aspect of the invention concerns the use of these antibodies in clinical application before, during or after transplant surgery, in bone marrow transplantation, in treatment of cancer (direct treatment of leukemic cells and indirect treatment of all types of cancer by activation of T-cell populations) and for therapeutic applications in immunoregulation.

The antibodies of the present invention are also useful in the detection of immunocompetent T-cells.

Additional aspects will become apparent upon study of the detailed description set forth below, which includes preferred embodiments of the present invention and which also includes other data for information purposes.

The description refers by way of example to the accompanying drawings, wherein:
Figure 1 graphically depicts the construction scheme of the BMA 031 genomic library;
Figure 2 describes the probes used to screen the library;
Figure 3 graphically sets forth, in linear fashion, the human constant region expression vectors;
Figures 4A, 4B and 4C show results of competitive immunofluorescence assays with BMA 031 Chimeric Antibodies.
Figures 5A and 5B show results of T-cell proliferation assays with BMA 031 Chimeric Antibodies.
Figures 6A, 6B and 6C show results of ADCC assays with the BMA 031 Chimeric Antibodies.
Figure 7 shows the results of competitive immunofluoresence assays with BMA-EUC1V3 antibody.

As used herein, the term "epitope" refers to the structure recognized by the monoclonal antibody BMA 031 (this antibody has been discussed in *Transplantation Proceedings,* **Vol XX**, No 2, Suppl 2: 103-109 (April 1988) for example, which explains that BMA 031 has stimulatory features different from those of several CD3 antibodies), and is generally thought to be independent of the remaining portion of the antigen on which the epitope is located. It is presently unknown exactly how the epitope is formed structurally but it is anticipated that it may be formed by either (i) a part of the amino acid sequence of the antigen molecule; (ii) the three-dimensional structure formed by non-contiguous amino acids within the same molecule; (iii) the three-dimensional structure formed by various molecules within an antigen complex; or (iv) some combination thereof. As used herein the term "monoclonal antibody (mAb)" means an antibody composition having a substantially homogeneous antibody population. It is not intended to be limited as regards to the source of the antibody or the manner in which it is made and in the most preferred embodiments is intended to include recombinant methods of manufacture.

As used herein with respect to the exemplified BMA 031 antibody, the term "functional equivalent" means a monoclonal antibody that: (a) blocks the binding of BMA 031 and whose binding is blocked by bound BMA 031; (b) binds selectively to human T-cells having expressed the alpha/beta TCR but not having expressed TCR-gamma and/or delta-chains; (c) has one of the known isotypes; (d) binds to the same antigen as BMA 031 as determined by immunoprecipitation, western blotting or other biochemical analyses; (e) binds to the same antigen as determined by cells transfected by gene(s) for the alpha/beta TCR or segments thereof.

### Example 1. Immunization and cell fusion

Three Balb/c mice (female; age: 6-8 weeks) were twice immunized intraperitonally with 1.5 x 10⁶ E⁺-cells each. Peripheral blood human T-lymphocytes were separated by the E-rosette-technique (rosette-formation with sheep red blood cells - E⁺-cells) and were derived from the peripheral blood of a healthy donor whose donated blood routinely tested HIV-negative for more than one year after having given the blood for immunization purposes.

Three days after the last immunization, the spleens of all three mice were removed, a single cell suspension was prepared and the lymphocytes were fused with the murine myeloma cell P3X63-Ag8.653 (ATCC # CRL 1580) according to a standard fusion protocol. The myeloma cell P3X63-Ag8.653 is described as an immunoglobulin non-producer mutant derived from the original myeloma cell P3-X63-Ag8 (ATCC # TIB 9).

After fusion, cells (1 x 10⁶ cells/well) were cultured in the presence of HAT-medium (Dulbecco's modified Eagle's medium + 10% FCS ⁺0.1mM hypoxanthine, 0.4 µM aminopterin, 16 iµM thymidine) to select for hybridoma cells.

### Example 2. Isolation and characterization of the hybridoma clone BMA 031

Supernatants of growing hybridoma cells (fusion number BW 242) were harvested routinely and tested for the presence of murine immunoglobulins in an ELISA test-system designed to measure murine IgG quantitatively. At the same time, the supernatants were tested for antibodies with specificity for human lymphocyte cell surface antigens.

Within this selection process, single cells were picked from the original well (BW 242/1177) and were cultured separately. In subsequent steps these cells were repeatedly cloned by micromanipulation under microscopic control (BW 242/412). During three cloning cycles 100% of growing clones produced monoclonal antibodies with identical binding specificity and identical behavior with respect to biochemical criteria. One clone was selected and designated as BMA 031.

Extensive analyses were carried out to define the specificity and the functional properties of the hybridoma clone, BMA 031.

A master seed bank and a working cell bank were established starting with the hybridoma clone BMA 031. The two cell banks underwent extensive examinations to assure absence of contamination with pathogens (mycoplasmatic, bacterial and viral infections). In addition, starting from the master seed bank, experiments were carried out which showed that even after the 50th culture passage detectable variations in antibody specificity were neither measurable nor could non-producer mutants be detected when analysed by single cell cloning and by calculating antibody production rates in bulk culture.

### Example 3. Specificity of BMA 031

Monoclonal antibodies directed to lymphocyte cell surface antigens are usually characterized by a binding assay. To analyse the specificity of BMA 031, cytofluorometric assay systems were used predominantly. In particular, binding assays were carried out as described below.

### 3.1 Analyses of the binding of BMA 031 with peripheral blood leukocytes

### Comparison of mAb reactivity with reference mAbs

Defined subpopulations of PBLs were labelled either with a reference mAb and/or with BMA 031. If both mAbs have identical specificity they will stain identical populations of cells. In these studies, the reference mAbs used were those which had been previously characterized in the Workshops for Human Leukocyte Differentiation Antigens I., II. and III. (Paris, 1982; Boston, 1984; Oxford, 1986; Bernard, A.E.: Leukocyte Typing. Springer-Verlag (1984); Reinherz, E.L.E.: Leukocyte Typing II. Springer-Verlag (1986); McMichael, A.J.E.: Leukocyte Typing III. Oxford University Press (1987).

### Expression of the antigen on different leukocyte subpopulations

Peripheral blood leukocytes were analyzed on an Ortho Cytofluorograph 50H/2150 Computer-system modified for single-step analyses of whole blood. Cells were either directly labelled with BMA 031-FITC and/or with reference mAbs or alternatively stained in a second step with isothiocyanate-fluoresceinated rabbit anti-mouse IgG (Ig-F(ab')2-FITC). By using cytofluorometric assays, it was discovered that binding of BMA 031 was only detected on cells of the T-cell lineage which express the alpha/beta T-cell receptor. BMA 031 does not react with cells which express the gamma/delta T-cell receptor. BMA 031 can therefore effectively be used to discriminate between alpha/beta-TCR⁺ and gamma/delta-TCR⁺ cells. Presence or absence of distinct TCR chains reflect the status of T-cell differentiation during T-cell ontogeny.

In peripheral blood, molecules of the CD3-complex are predominantly expressed in association with the human alpha/beta-TCR (7). In healthy blood donors, the frequency of T-lymphocytes stained by BMA 031 is usually only 1-5% lower than that measured with CD3 mAbs (for normal frequencies of CD3⁺ cells see 1-3). As shown with cloned T-cells, this population of CD3⁺ BMA 031⁻ cells express the gamma/delta TCR instead of the alpha/beta TCR. In pathological situations, however, the frequency of CD3⁺ BMA 031⁻ T-cells can increase to 20% of the CD3⁺ cells in individual patients. On CD3⁺ BMA 031⁺ cells, binding of BMA 031-FITC is blocked by OKT3 and vice versa in competitive immunofluorescence assays. Nevertheless, by analyzing such data in more detail (comparison of fluorescence-histograms) and by blocking studies with anti-idiotypic antisera it can be clearly shown that BMA 031 recognizes a different epitope than all known CD3-mAbs.

### Example 4. Characterization of the functional properties of BMA 031

BMA 031 is a murine monoclonal antibody of the IgG2b isotype. Due to the unique specificity and the isotype of BMA 031, this antibody triggers a specific pattern of biological functions. After binding to T-cells, BMA 031 does not induce T-cell proliferation comparable to CD3-mAbs of the IgG2a isotype (e.g. BMA 030, OKT3) in a three day thymidine incorporation assay nor does it induce Ca²⁺ influx in resting T-lymphocytes . In contrast to CD3-mAbs such as BMA 030 or OKT3, binding of BMA 031 to T-cells induces antigen modulation only weakly and triggers the release/production of cytokines to a very low extent.

### Example 5. Preparation of DNA and RNA from BMA 031

For preparation of genomic DNA, approximately 1 x 10⁹ cells were grown in T-flasks. DNA was prepared by lysis in SDS, digestion with proteinase K and RNAse A and sequential, gentle, phenol/chloroform extractions in high salt. Low density agarose gels indicated that the average length of the genomic DNA was greater than 50 kilobases (Kb); long length is important in creation of complete genomic clone libraries in lambda phage vectors. The yield was about 10 mg of DNA.

Approximately 10⁹ cells were also grown for RNA isolation. Cells were lysed and RNA extracted using guanidinium thiocyanate. RNA yield was about 10 mg and it appeared clean and undegraded on agarose gels. Poly A⁺ RNA was prepared by binding total RNA to oligo dT cellulose. Yield was about 5%, i.e. about 500 µg of poly A⁺ mRNA.

### Example 6. Sequencing BMA 031 heavy (H) and light (L) chains by primer extension of mRNA

Because of the high abundance of specific mRNA, immunoglobulins can be sequenced by primer extension of total poly A⁺ mRNA. This is also facilitated by their common constant regions which allow synthesis of "universal" primers with which to begin the extension. Subsequent primers can be made as sequence information is gathered. "Universal" H and L chain primers (Light chain "Universal" Primer: ⁵'TGGATGGTGGGAAGATG³'; Heavy chain "Universal" Primer: ⁵'GGGGGCCAGTGGATAGAC³') were isotopically end labelled with polynucleotide kinase and hybridized individually to about 7 µg aliquots of BMA 031 poly A⁺ mRNA. The hybridized product was extended using avian reverse transcriptase in the presence of dideoxy nucleoside triphosphates. Reaction products were separated on gradient acrylamide gels and autoradiographed. About 200 nucleotides (NT) of sequence were read from the initial extension. For both H and L chains, two additional primers were synthesized, each 17-18 NT in length, namely

#149 (Light) 5'AGGGACTCCAGAAGCCA3'
#180 (Light) 5'CTGGAGATGCAACATG3'
#179 (Heavy) 5'CTCCATGTAGGCTGTACT3'
#178 (Heavy) 5'CCAGAAGCCTTGCAGGA3'

The extension primers as described above generate complete, overlapping sequence (a total of about 440 NT for each gene) specifying the H and L chain variable (V) regions.

The sequences obtained are listed in Table 1A (Heavy) and 1B (Light). Positions of the signal sequence, start of the mature protein, invariant cysteines, complementarity determining regions (CDR), joining region (J), and primers used in sequencing are indicated. These sequences were confirmed by DNA sequencing of isolated lambda clones (see Example 7 below).

### Example 7. Construction and Screening of a Genomic Clone library

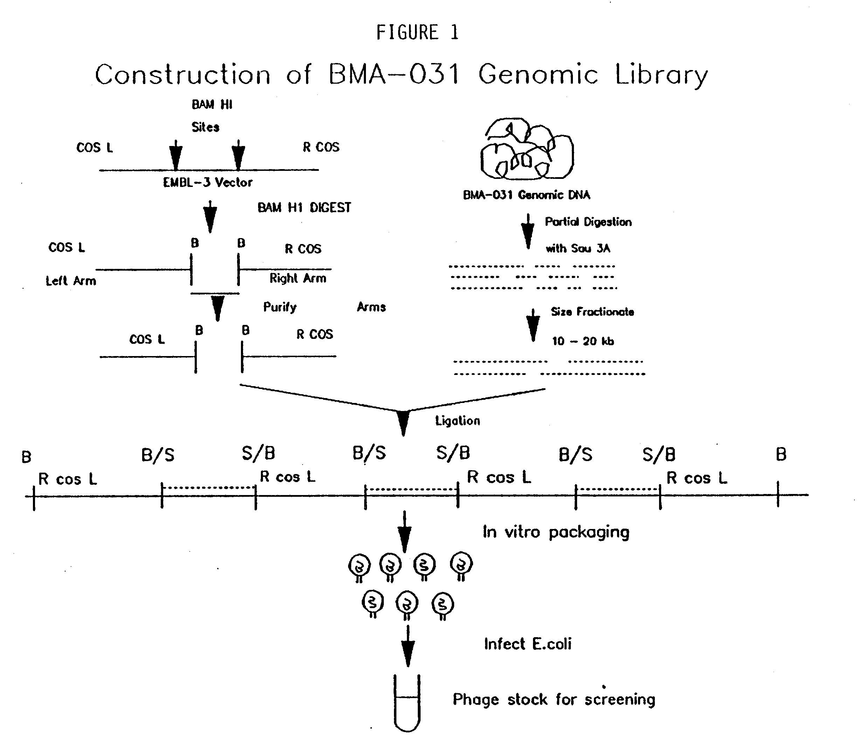

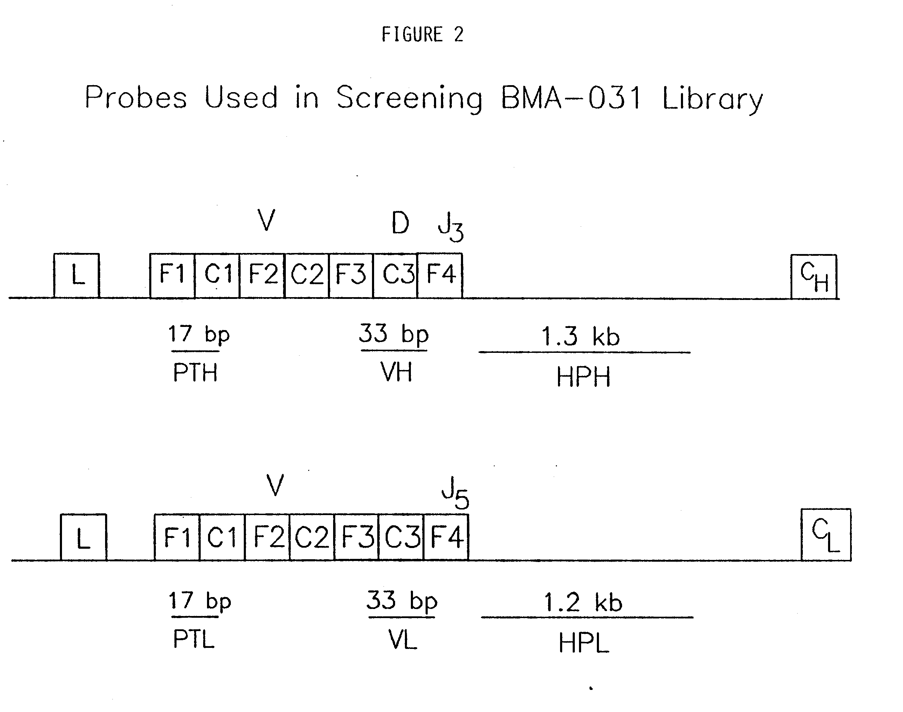

The general strategy of the library construction is depicted in Figure 1 and Table 2 Parts A and B. Total genomic DNA was partially digested with the restriction enzyme Sau 3A. DNA from the digest in the size range 10-20 Kb was isolated by preparative gel electrophoresis and binding to glass-impregnated paper. The resulting material was ligated with phage "arms" from the lambda vector EMBL-3 (ATCC #37266). Ligated material was packaged into phage particles (cloning efficiency 1 x 10⁶ pfu/µg genomic DNA). Recombinant clones were plated at a density of 10⁵/plate. Replica filter lifts were performed on each plate and the nitrocellulose filter discs were processed for hybridization. These disks were hybridized in duplicate to ³²p-labelled fragment probes derived from the intron between V and C exons of H or L chain (these probes are "universal" Ig probes). Putative positive clones were confirmed and plaque-purified by up to four rounds of rescreening. Rescreening was carried out with radiolabelled, synthesized oligomers (17 - 33 NT) corresponding to hypervariable regions known from Example 6. The approximate relative location of the fragments and oligomers used as probes for screening are indicated in Figure 2.

After exhaustive screening of the library, multiple positive lambda clones were isolated containing BMA 031 H and L chain exons. As listed in Table 2, Part C, of 14 H chain clones initially identified, 4 were true positives; of 27 L chain clones, 7 were real. The genomic sequence of the H and L chains as determined by both Sanger and Maxam-Gilbert sequencing, are listed in Tables 3A and 3B, respectively.

### Example 8. Vector construction for expression of chimeric BMA 031

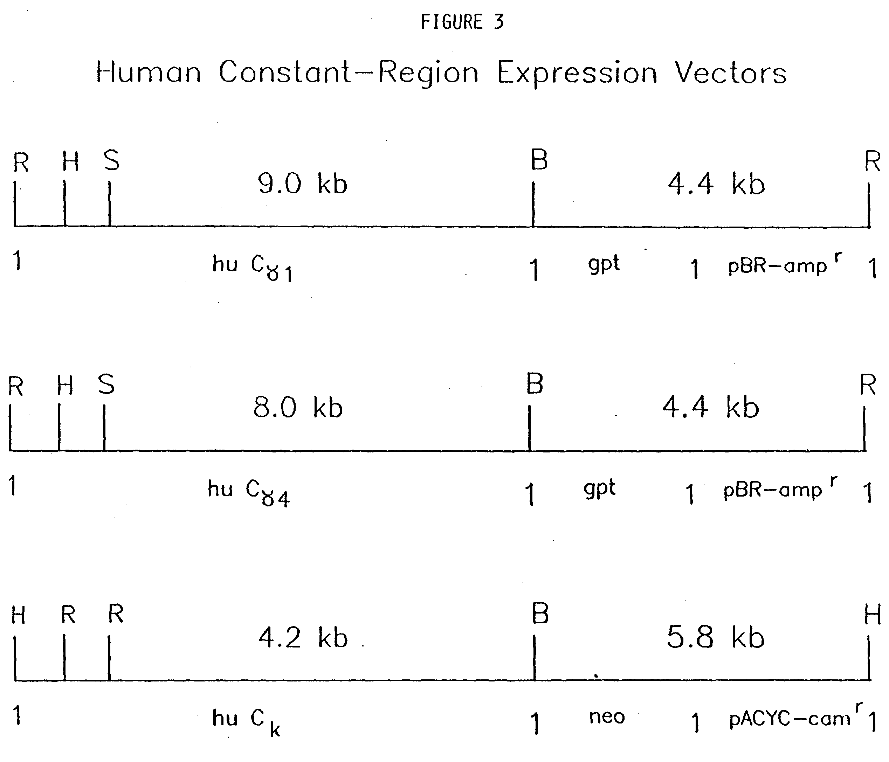

The H chain of chimeric BMA 031 was synthesized by a vector containing the human gamma 1 constant region or a second vector containing the gamma 4 constant region. For the L chain, the vector contained the human kappa constant exon. Each vector also contained an upstream cloning site for the respective BMA V gene, drug selectable markers, and signals necessary to allow replication in bacteria. Restriction maps of these vectors are shown in Figure 3. The V exons of BMA 031 H and L chains were subcloned into the vectors described above.

### Example 9. Transfection of chimeric BMA 031 gene into myeloma cells for expression

The chimeric constructs described above were co-transfected into SP2/0 (ATCC #CRL 1581), a non-immunoglobulin-producing murine hybridoma, by electroporation. After a 48 hour expression period, the resultant cells or, transfectomas, were placed in media containing both mycophenolic acid (1 µg/ml) and Geneticin (1 mg/ml) (Gibco). Growth of the transfectomas was apparent in about two weeks. The transfection efficiency in double selection was approximately 1 x 10⁻⁵. The proportion of drug-resistant clones secreting antibody was greater than 50% with the level of antibody secretion varying from about 1 µg/ml to about 17 µg/ml.

### Example 10. Subcloning of Transfectomas

The BMA 031-G1 and BMA 031-G4 cell lines were subcloned to eliminate genetic heterogeneity that may have arisen since the original transfection. The best subclone of each chimeric transfectoma was isolated and analyzed for antibody production. These final cell lines, BMA031-G1-1 and BMA 031-G4-1, produced antibody at a rate of 7 pg/cell/24 hours for IgG1 and 5 pg/cell/24 hours or IgG4. Saturated cultures accumulate antibody to 35 µg/ml for IgG1 and 15 µg/ml for IgG4.

### Example 11. Characterization of chimeric BMA 031

The antibodies, herein referred to as BMA 031-G1 and BMA 031-G4, secreted by BMA 031-G1-1 and BMA 031-G4-1, respectively, were tested to ensure that they were indeed BMA 031 mouse-human chimeric antibodies. Analysis by a series of ELISA assays showed that the antibodies contained human kappa and human gamma constant regions. Moreover, the antibodies did not react with antibodies directed against murine kappa or gamma constant regions. Isotyping reagents also confirmed that the chimeric antibodies were of the IgG1 and IgG4 isotypes.

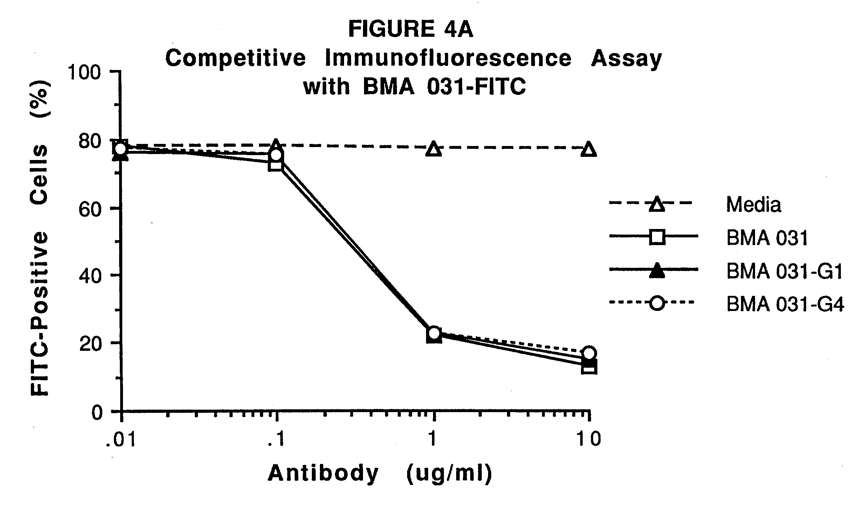

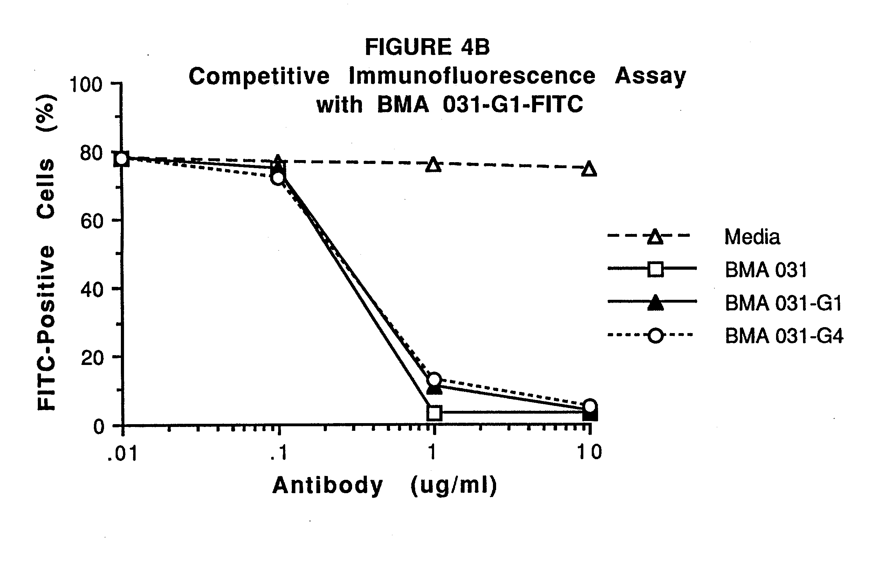

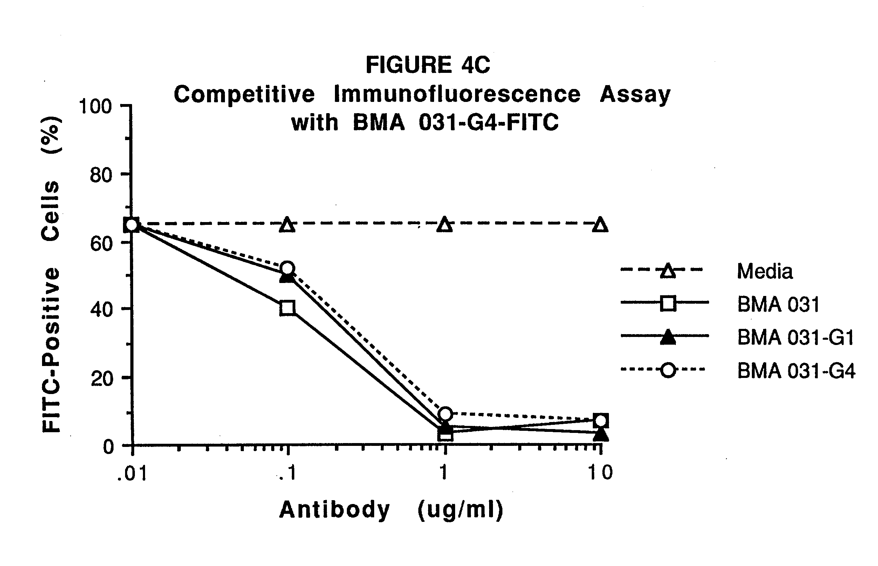

The purified chimeric antibodies were also shown to be functionally active in an immobilized cell ELISA assay. Both chimeric antibodies bound to PBL-ALL cells in a similar fashion as the original murine BMA 031. Directly labelled chimeric antibodies BMA 031-G1-FITC, and BMA 031-G4-FITC, were used in cytofluorometric assays systems to compare the specificity of these antibodies with murine BMA 031-FITC. A typical reactivity pattern of these antibodies with peripheral blood leukocytes is shown in Table 4. In competitive immunofluorescence assays, preincubation of human lymphocytes with chimeric BMA 031-G1 or BMA 031-G4 blocked binding of murine BMA 031-FITC and vice versa (See Figure 4A-4C). All these data indicate that the specificity of chimeric BMA 031-G1 and BMA 031-G4 is identical to murine BMA 031.

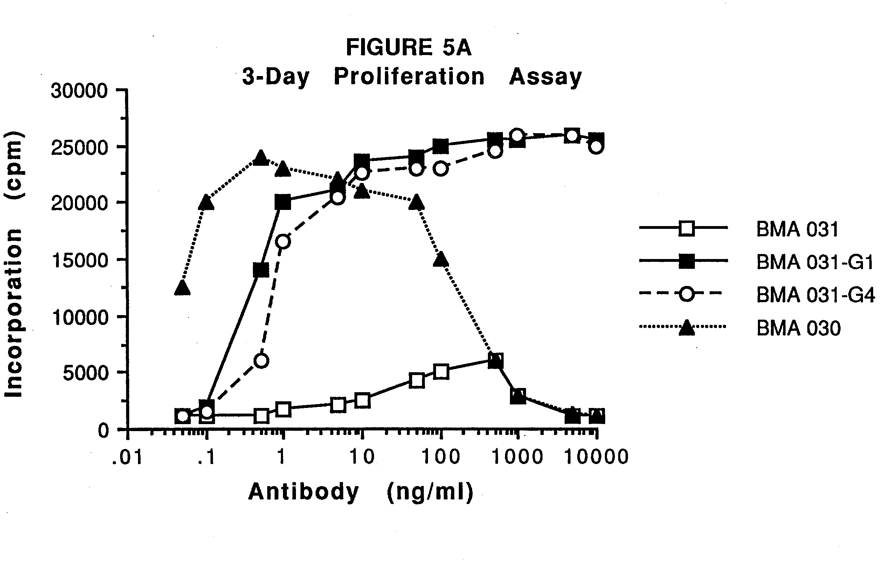

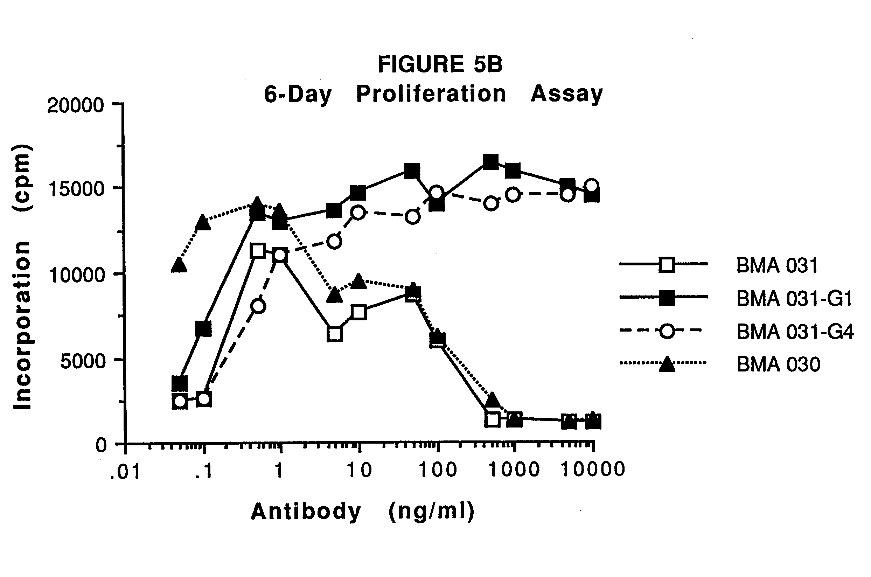

However, the functional properties of chimeric BMA 031-antibodies clearly differ from murine BMA 031 or from CD3-mAbs such as BMA 030. It is well known that most CD3-mAbs (e.g. BMA 030) trigger T-cell proliferation even in low doses but have no mitogenic effect when used in high concentrations (See Figures 5A and 5B). This effect is known as "high dose inhibition".

In contrast to murine BMA 031, both chimeric BMA 031-mAbs are highly effective in triggering T-lymphocyte proliferation as measured in a 3 day or 6 day thymidine incorporation assay without showing any evidence of high dose inhibition effects even in much higher dose ranges. Two representative experiments are shown in Figures 5A and 5B. In such experiments, the height of responsiveness and the optimal concentration for stimulation with BMA 030 or other CD3-mAbs may differ from blood donor to blood donor due to individual differences of the immune status.

As described above (Example 4), in contrast to CD3-mAbs, binding of murine BMA 031 to human T-lymphocytes usually results in the release of only low concentrations of immunomediators. Again, chimeric BMA 031 antibodies differ in this respect from murine BMA 031 as well as from BMA 030 (CD3). In Table 5, a representative experiment is shown, where release of immunomediators is measured by induction of HLA-Dr expression on COLO 205-cells.

It is known from the literature that COLO 205 cells respond with an enhanced expression of HLA-DR antigens after incubation with recombinant immunomediators like gamma-Interferon, Tumor Necrosis Factor or supernatants of activated T-cells which contain such factors. By comparing the enhancement of HLA-DR expression induced by supernatants of activated T-cells with reference values generated by the use of recombinant factors such as gamma-Interferon, the amount of immunomediators in culture supernatants can be determined. The data summarized in Table 5 indicate that the kinetics and the amount of immunomediators released after binding of chimeric BMA 031 antibodies differ from the effects triggered by murine BMA 031 or the CD3-mAb BMA 030.

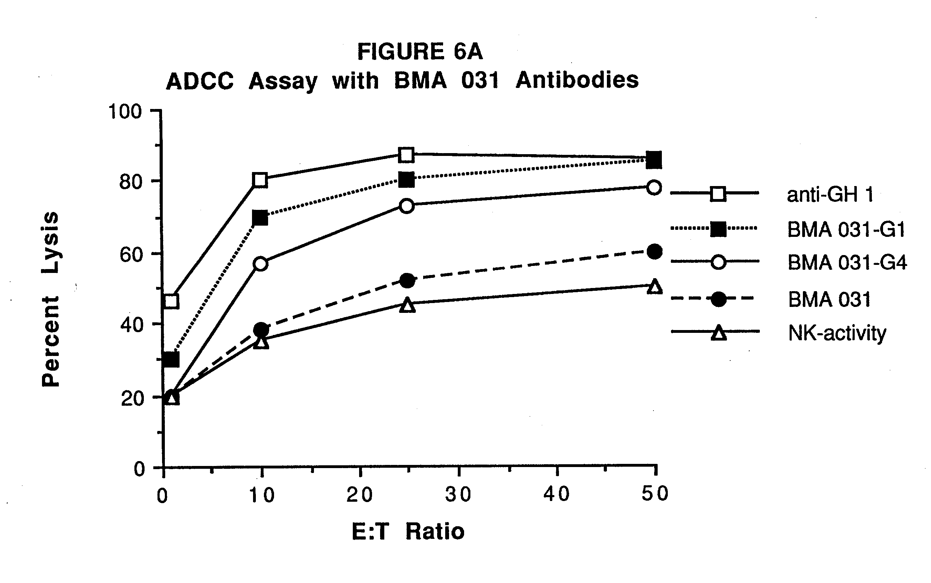

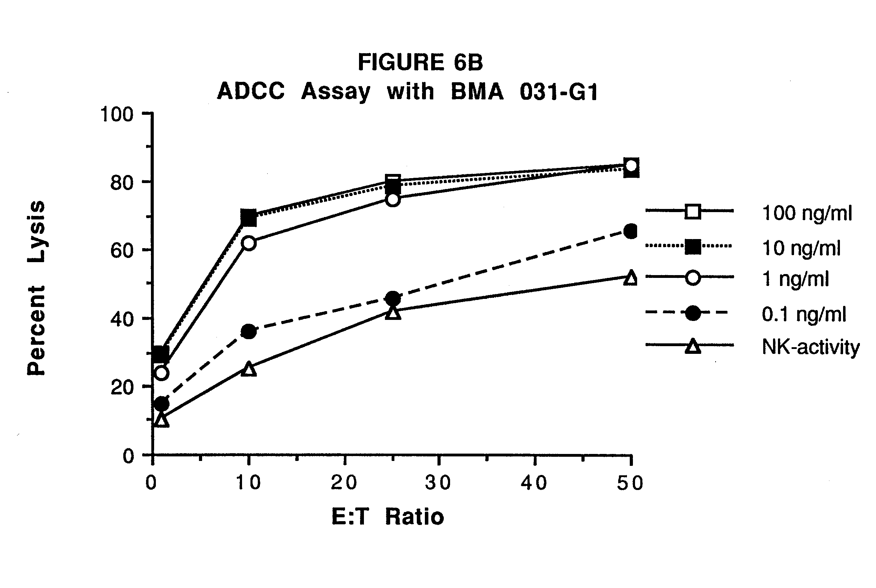

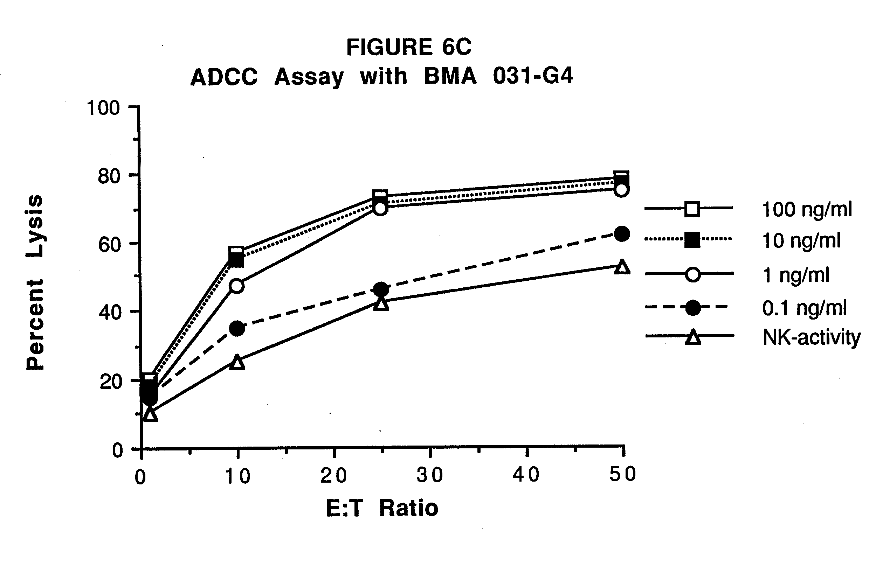

Since the chimeric BMA 031 antibodies were able to interact efficiently with human Fc receptors in the T cell proliferation assays, there was a strong possibility that they would have high ADCC titers as well. To evaluate the ADCC capacity of these mAbs, they were compared to rabbit anti-GH-1 antiserum selected as the best out of eight rabbit anti-human T cell globulins in ADCC capacity. The data of a representative experiment is shown in Figures 6A-6C. Even at low effector:target cell ratios (Figure 6A) or extremely low antibody concentrations (Figure 6B, 6C), chimeric BMA 031 antibodies are highly potent in killing HPB-ALL cells. In contrast, murine BMA 031 is very poor at ADCC. Accordingly, if cytolysis is an important criteria, BMA 031 can be advantageously used conjugated to a cytotoxic moiety.

### Example 12. Production of "civilized" BMA 031 Antibodies

### Determination of civilized BMA 031 amino acid sequence

In the past, "humanization" has been associated with chimeric constructions in which murine V regions are expressed with human C regions. To avoid confusion, the term "civilized" is used herein for constructions of "humanized" V regions expressed with human C regions.

To determine the optimal human sequence with which to civilize the murine BMA 031 antibody, the murine BMA 031 amino acid sequence was used to search the NBRF data base for the most homologous human antibody. Since molecular models of antibodies show strong interactions between the heavy and light chains, it was decided to use the heavy and light chains from the same human antibody. The human EU antibody turned out to be the best overall choice. The homology between the BMA 031 and EU FRs (#1-3) was 66% for the H chain and 63% for the L chain. The BMA 031 antibody uses JH3 and Jκ5. These are most homologous to human JH4 and Jκ4. A first generation civilized BMA 031 antibody would contain BMA 031 CDRs, EU FRs and homolgous human J regions. This antibody is referred to as BMA 031-EUCIV1 (Table 6A and 6B).

A refinement to this basic civilized version can advantageously be made in the sequence immediately before and after the CDRs. The CDRs are assigned based on sequence homology data (Kabat et al., 1987). Molecular models of antibodies have shown that the actual CDR loops can contain amino acids up to 4 amino acids away from the "Kabat" CDRs. Therefore, maintaining at least the major amino acid differences (in size or charge) within 4 amino acids of the CDRs as murine may be beneficial. This antibody is referred to as BMA 031-EUCIV2 (Table 6A and 6B). Additionally, all differences within four amino acids of the CDRs could be maintained murine. This antibody has been designated as BMA 031-EUCIV3.

It will be readily recognized that further refinements can be made, but, without complex computer modelling, it is difficult to prioritize their importance. However, a simplified computer model was generated based on sequence homology to other antibodies with solved structures. This model was used to judge proximity of framework amino acids to the CDRs. The results of this analysis are shown in Tables 7A and 7B. These results were used to address the refinements discussed below. For example, several amino acids are either BMA 031 specific or EU specific (ie. different from the consensus sequence within their subgroups). Since these amino acids presumably arose through somatic mutation to enhance their respective activities, it would seem logical to maintain the BMA 031 specific amino acids and change the EU specific amino acids to the human consensus. But this can have potential adverse consequences. Changing an amino acid in one chain may cause changes in the interactions with other amino acids of that chain as well as with amino acids in the other chain. Therefore, extreme caution must be exercised to limit the number of changes. Table 8 outlines these potential changes.

As can be seen, EU differs from the human VH-I subgroup consensus sequence in six positions. Three are within 4 amino acids of the CDRs (#70,95,98), and these are addressed in BMA-EUCIV3. In one position (#93) the human consensus sequence is the same as BMA 031. One could rationalize changing this from EU back to BMA 031, so this change was incorporated into BMA-EUCIV3. For the two remaining positions (#72,74), there is no human consensus. However, the computer model shows that they may be close to the CDRs so it was decided to use the BMA 031 amino acids in BMA-EUCIV4. The light chain had five EU specific amino acids. One is within 4 amino acids of the CDRs (#48) and is maintained as BMA 031 in BMA-EUCIV3. In two positions (#63,81) the human consensus is the same as BMA 031 and therefore could be maintained as BMA 031. These changes were made in BMA-EUCIV4. The other two positions (#10,70) are open to debate. The computer model identified position #70 as being potentially important so this change was made in BMA-EUCIV4. Amino acid #10 was kept as EU. There are eight BMA 031 specific amino acids in the H chain. In two positions (#7,82) the BMA 031 sequence is the same as EU. Position #72 was addressed above. His₉₄ is unique to BMA 031 and very close to CDR3; it was decided to incorporate this change into BMA-EUCIV3. Of the remaining four positions (#1,9,20,40), only #40 is close to the CDRs so this change was made in BMA-EUCIV4. The others were maintained EU. There are no BMA 031 specific amino acids in the L chain. The sequence is identical to the subgroup VI consensus.

The final sequence of BMA-EUCIV4 was determined based on the preliminary computer model. For the heavy chain, two additional positions appear to be close to the CDRs, #77 and #87, and these were changed to the BMA 031 amino acids. For the light chain, two positions (#21,60) appeared to be removed from the CDRs; we decided to use the EU amino acids in this variant. Five additional positions (#1,3,4,42,71,100) were judged to be close enough to the CDRs for interaction, so they were changed to the BMA 031 amino acids.

### Synthesis and expression of civilized BMA 031 antibodies

The light and heavy chain variable region exons encoding the civilized antibodies were synthesized and replaced into the previously isolated genomic fragments of BMA 031. These fragments were inserted into mammalian expression vectors containing the human kappa and gamma 1 constant region exons. The civilized genes were transfected into Sp2/0 hybridoma cells by electroporation and transfectomas secreting civilized BMA 031 were isolated. Secretion levels varied up to 7 pg/cell/24 hr.

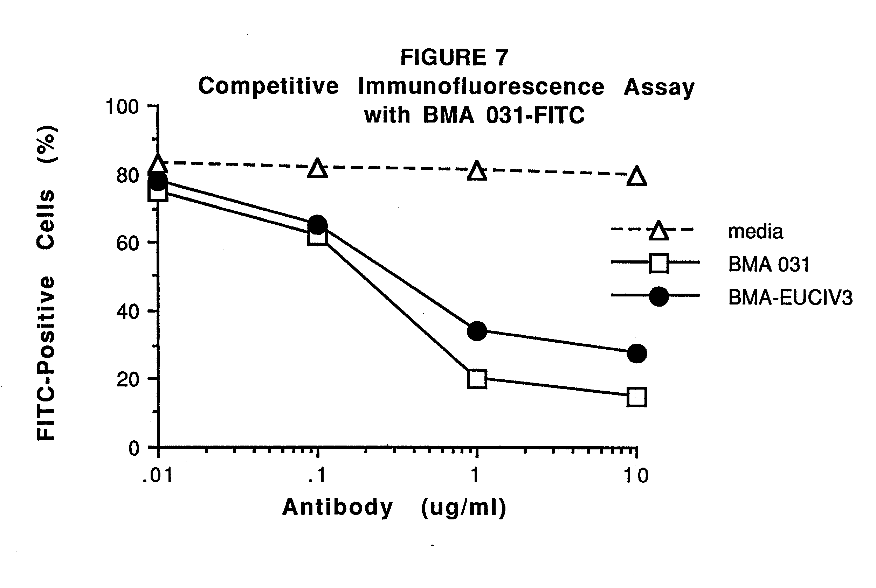

The BMA-EUCIV1 and BMA-EUCIV2 antibodies were unable to bind to T cells. In contrast, BMA-EUCIV3 bound specifically to T cells and competed effectively with both the murine BMA 031 antibody and the previously constructed chimeric BMA 031-G1 antibody for binding to these cells (Figure 7).

### Framework amino acids important for antigen binding

The T cell binding data with the civilized BMA 031 antibodies show the importance of framework amino acids in antigen binding. Inclusion of only the BMA 031 CDRs (BMA-EUCIV1) was not sufficient to maintain affinity for antigen. Twelve amino acid substitutions were made in the heavy chain V region to regain binding affinity (#27,28,30,38,48,67,68,70,93,94,95,98). Of these, six may be more important (#38,48,70,93,94,95) since they represent changes from BMA-EUCIV2, which does not bind well, to BMA-EUCIV3, which does bind well. Similarly, for the light chain V region, five amino acid substitutions were made (#21,46,47,48,60). Of these, three (#21,47,48) were made from BMA-EUCIV2 to BMA-EUCIV3 and thus may be more important.

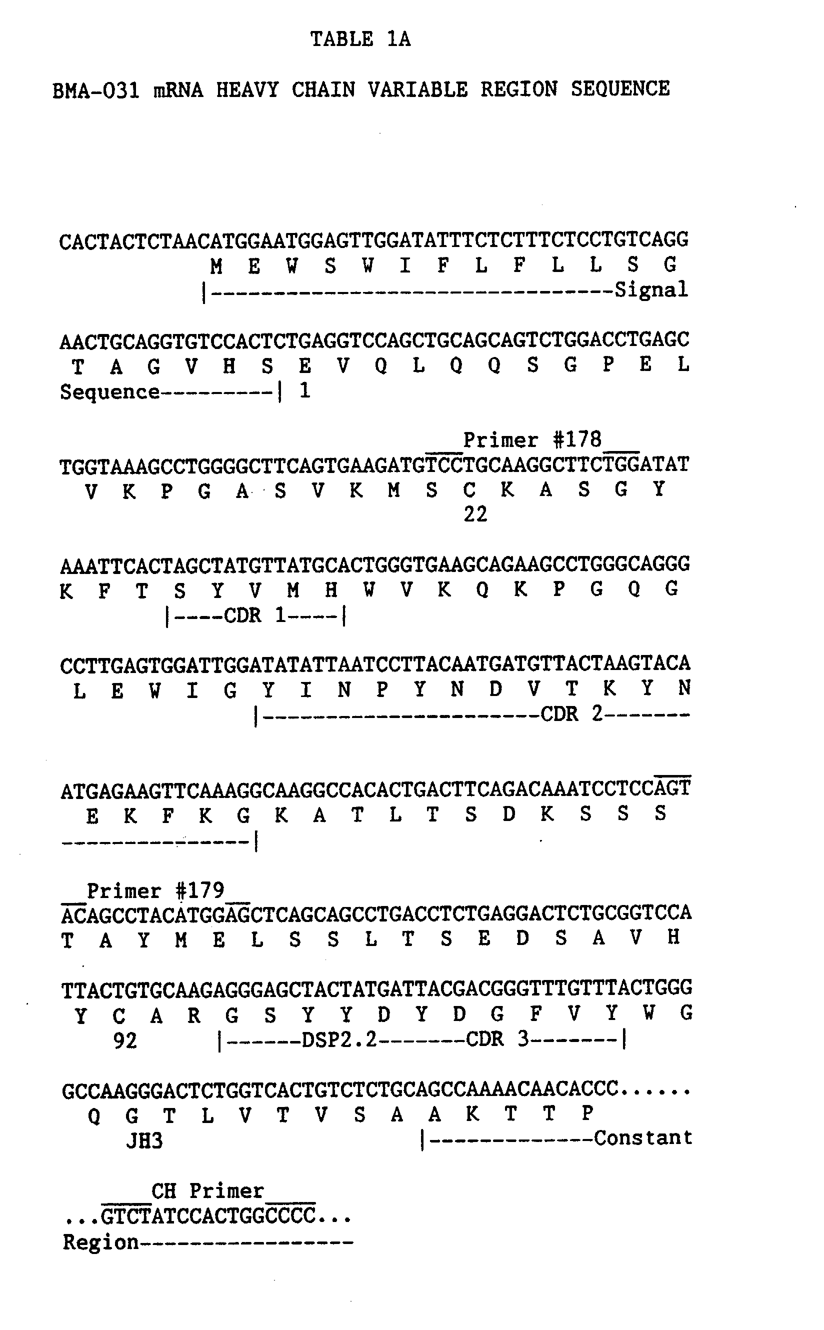

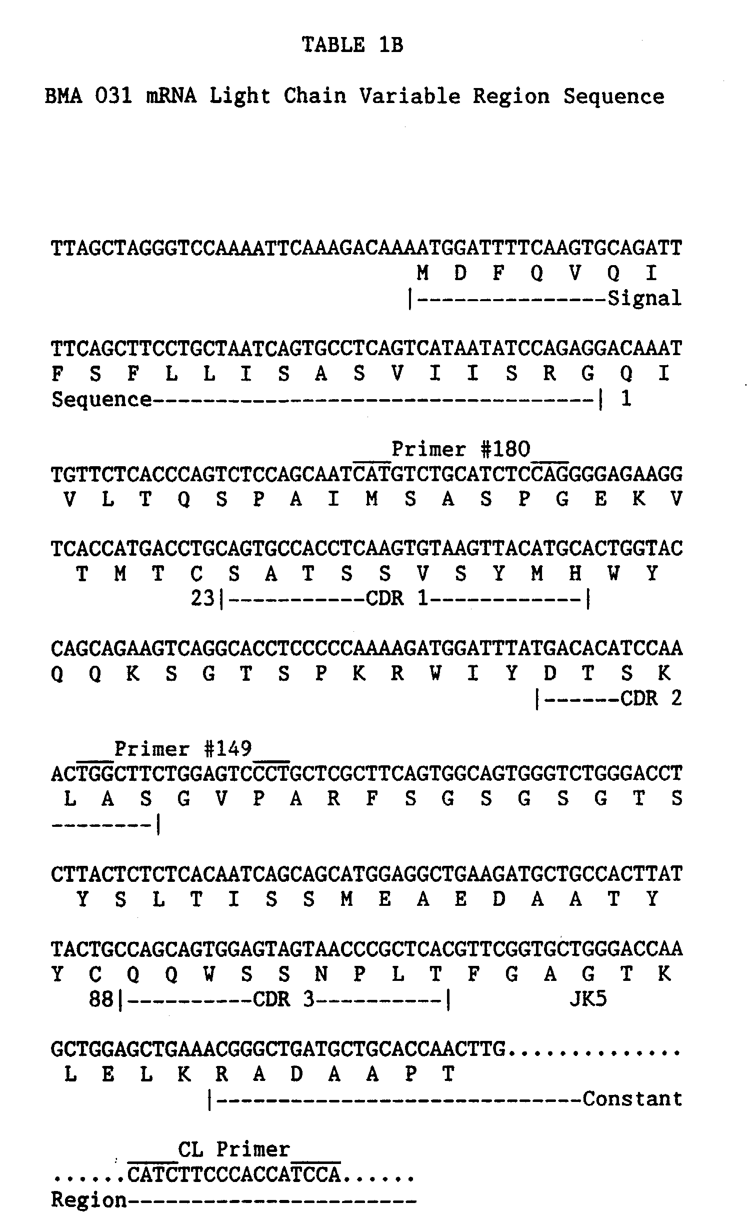

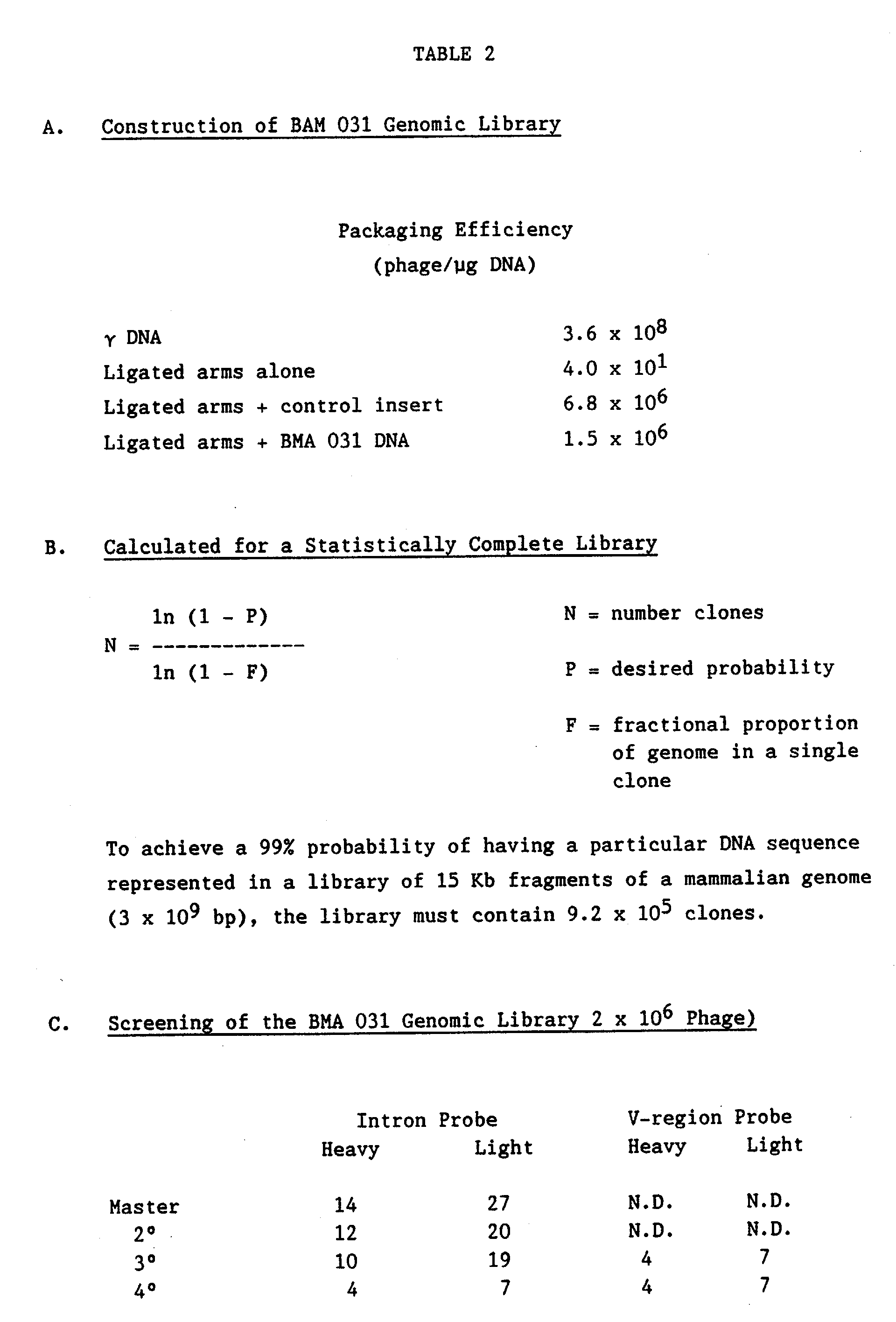

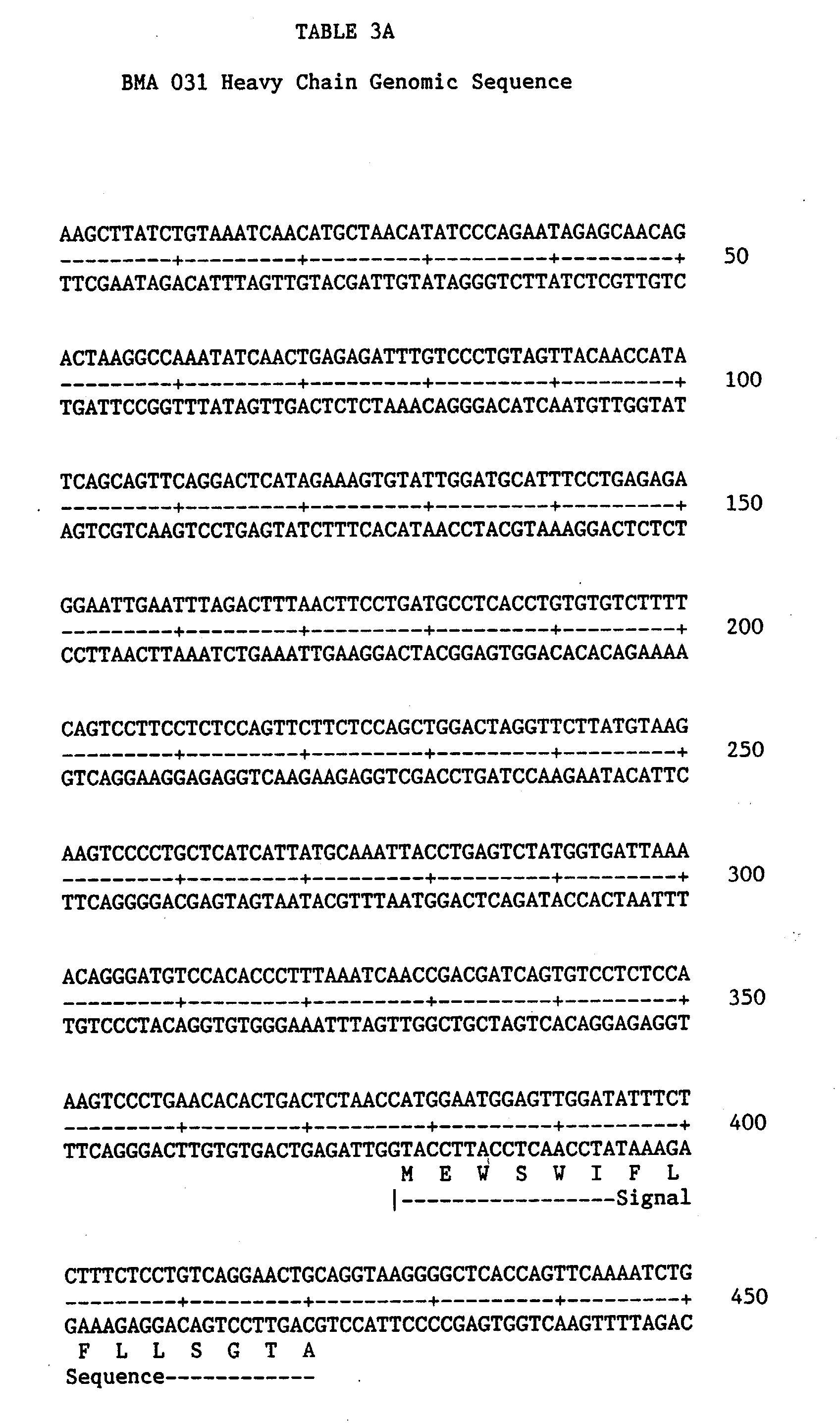

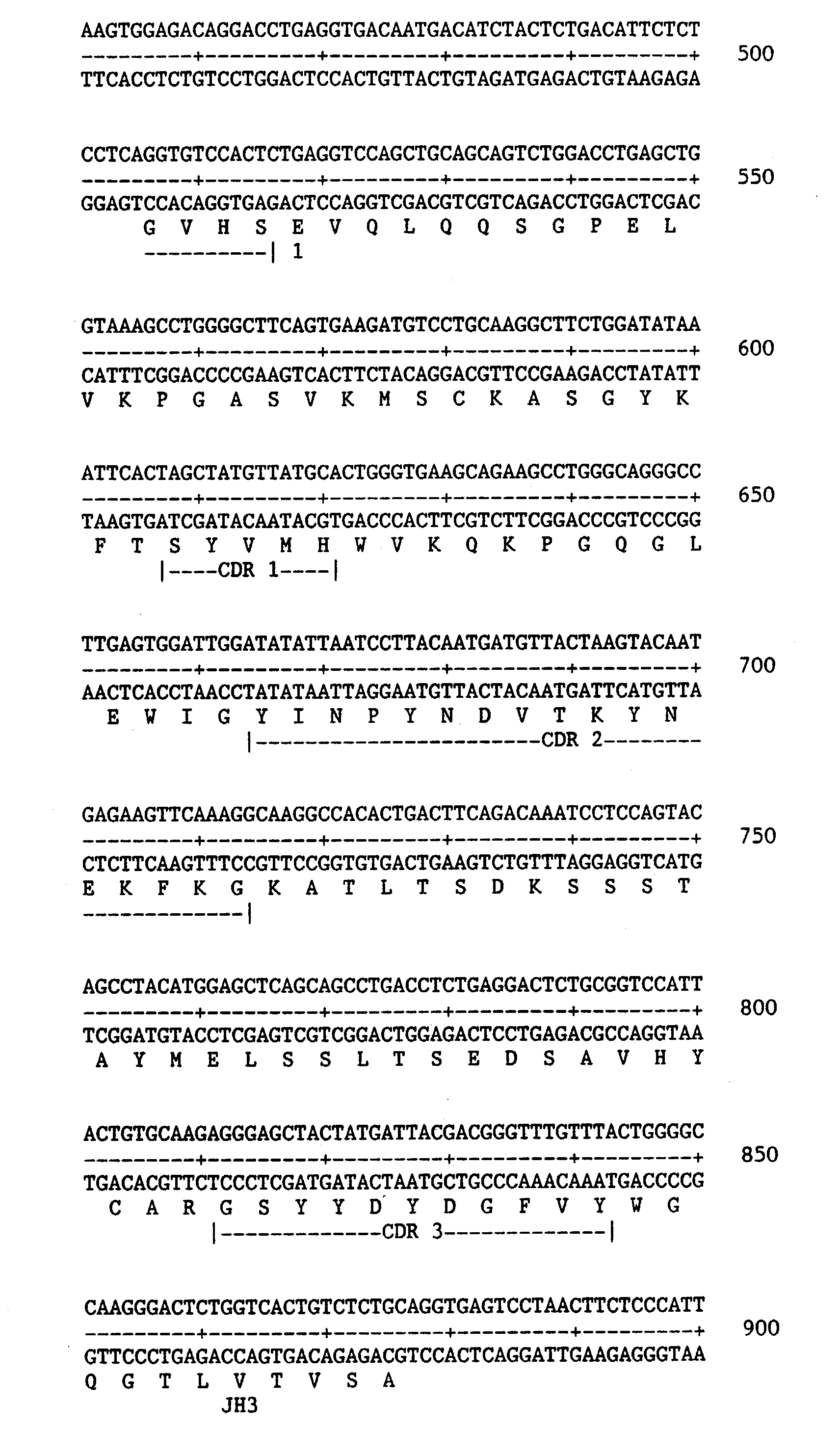

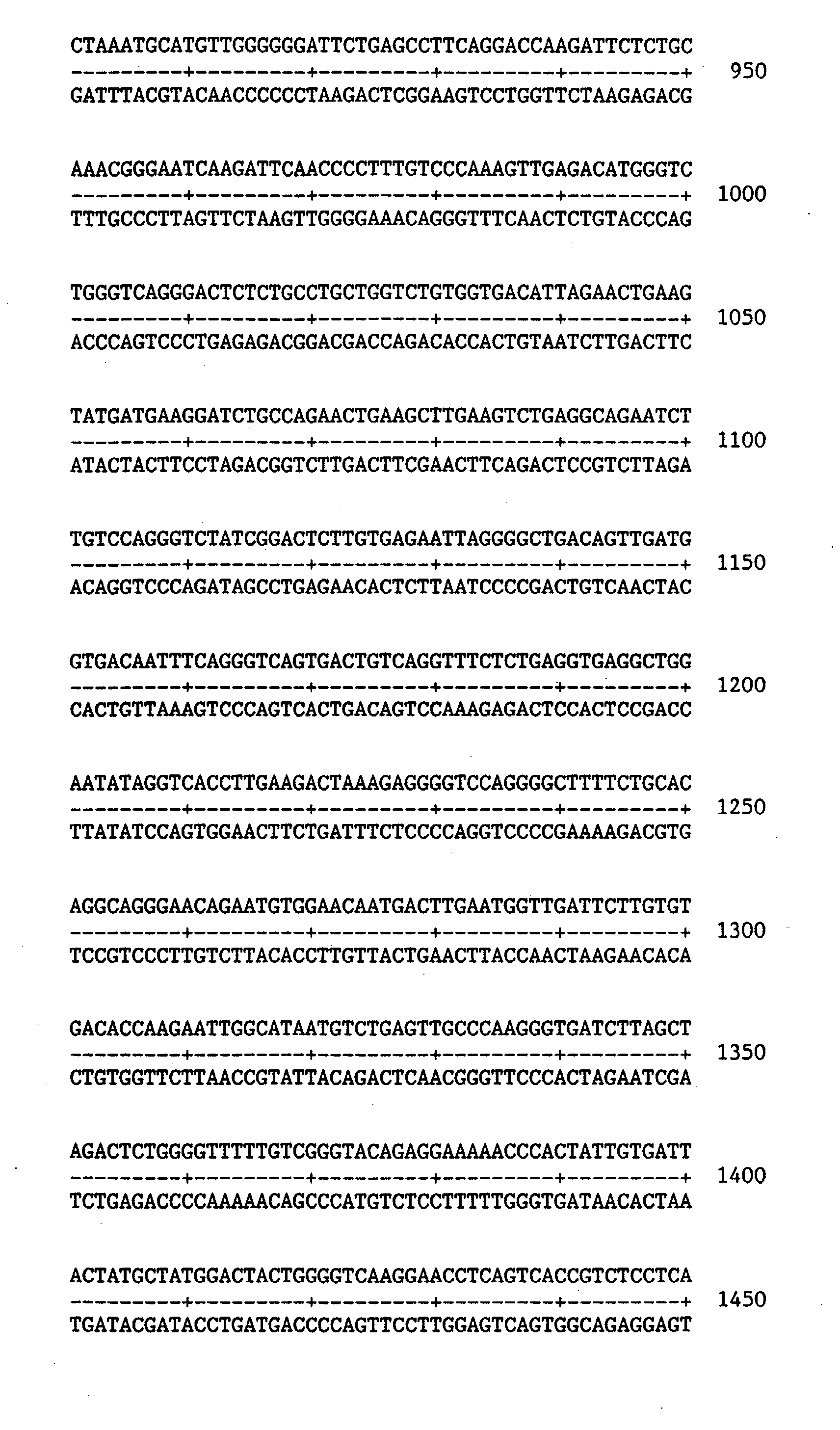

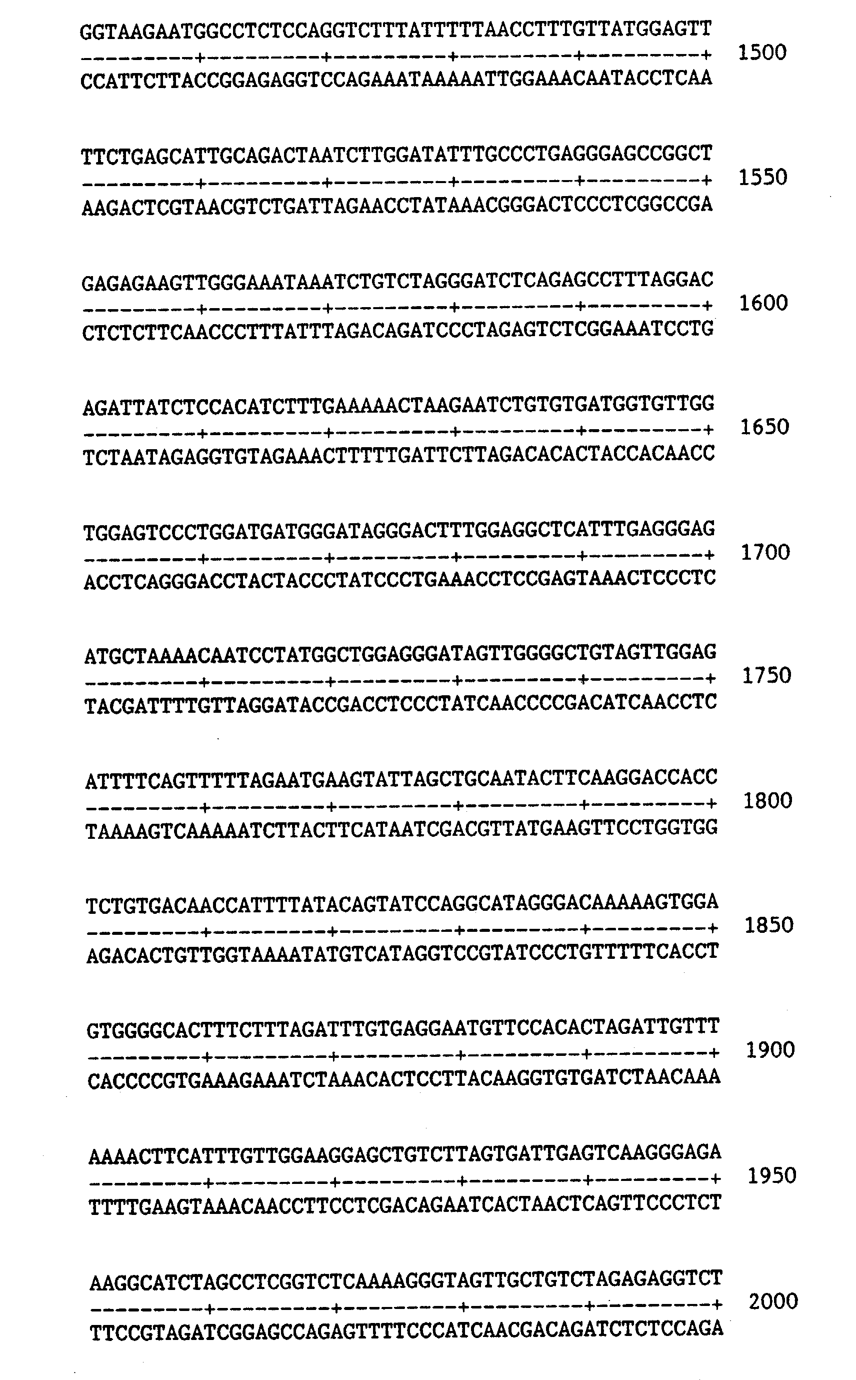

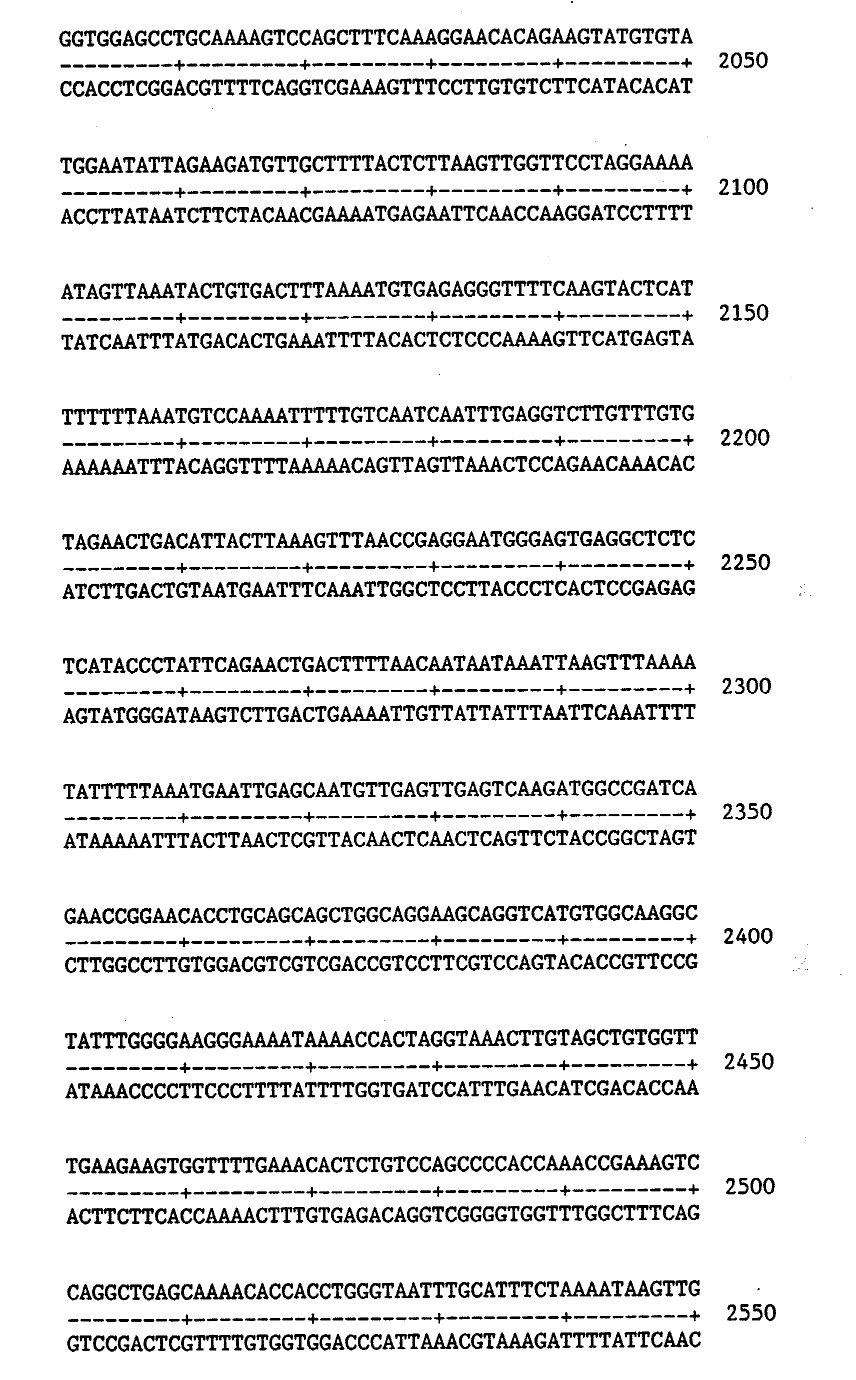

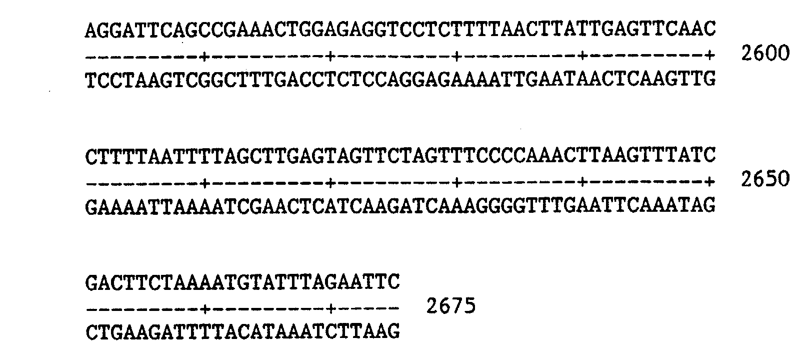

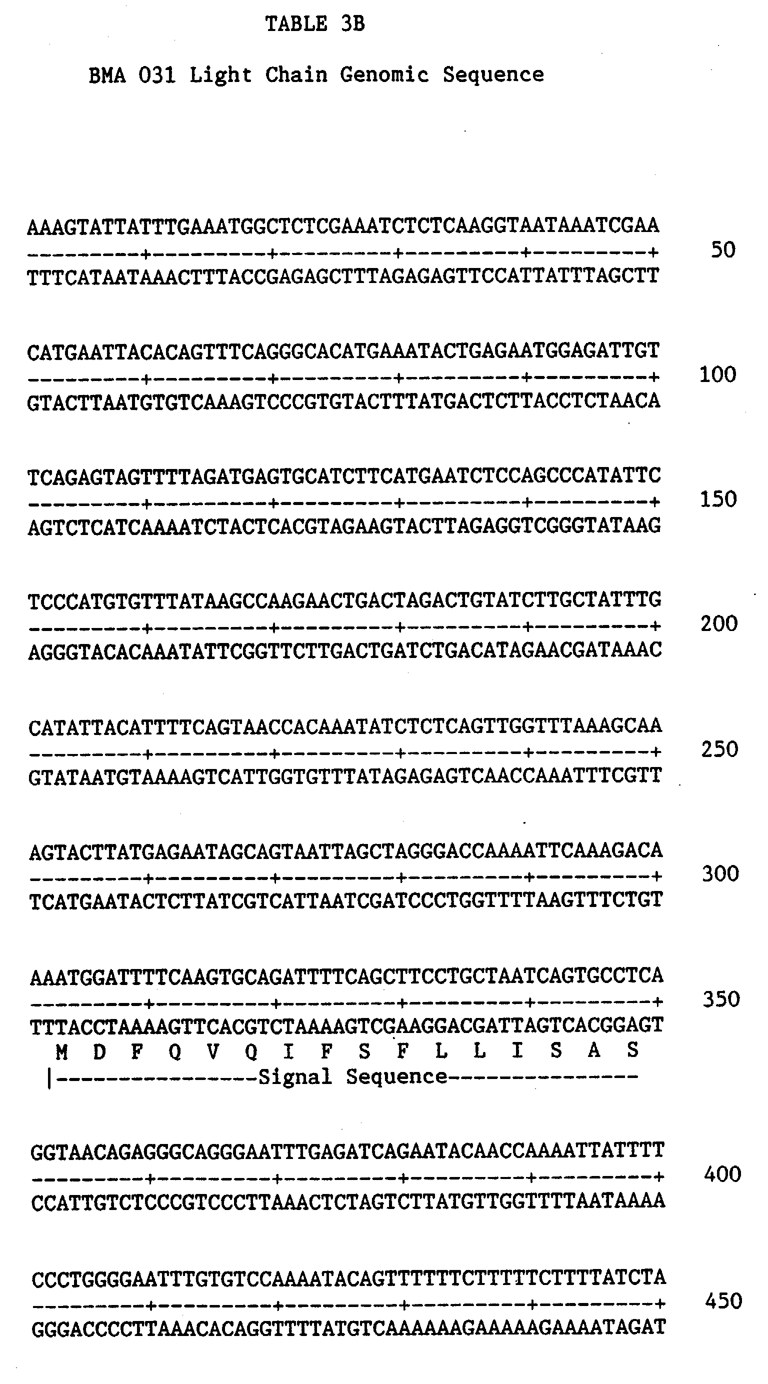

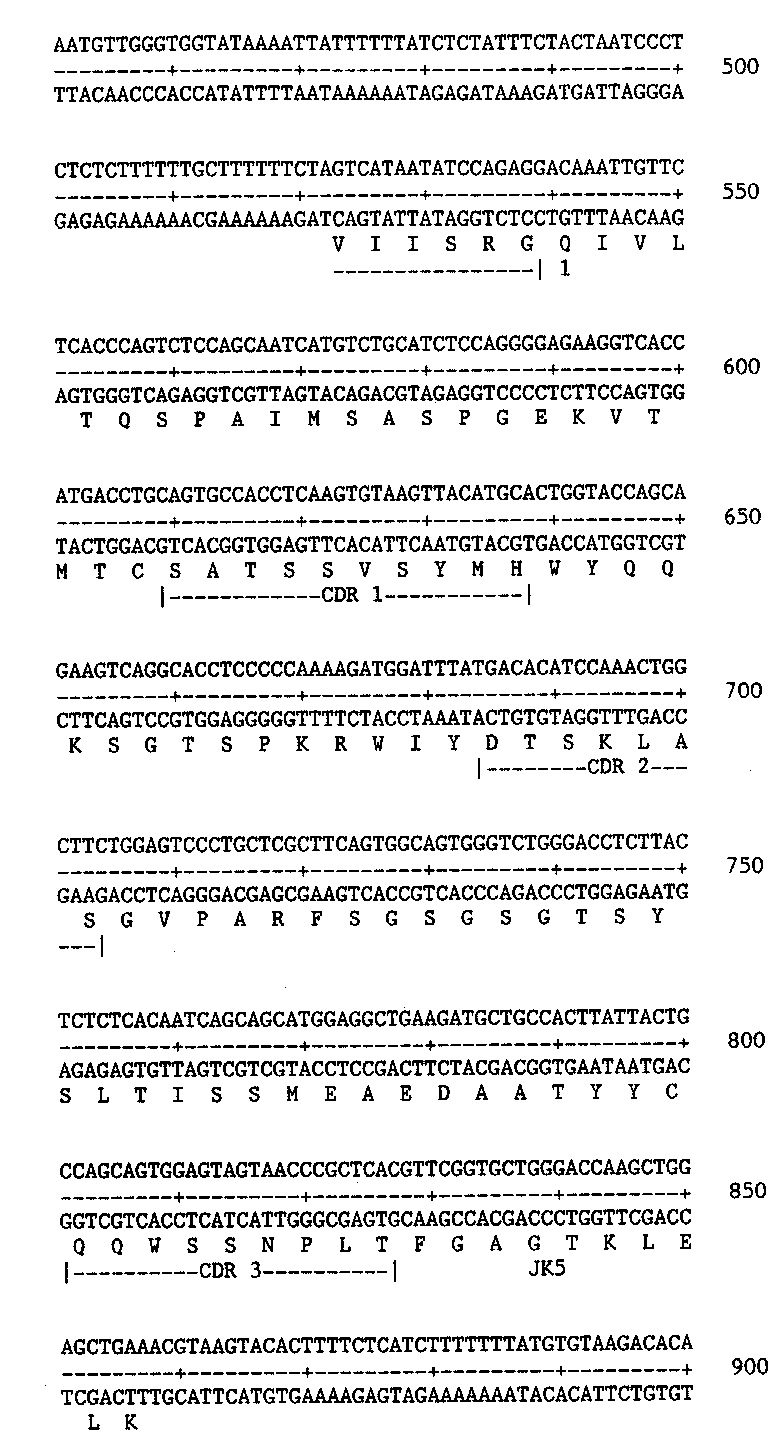

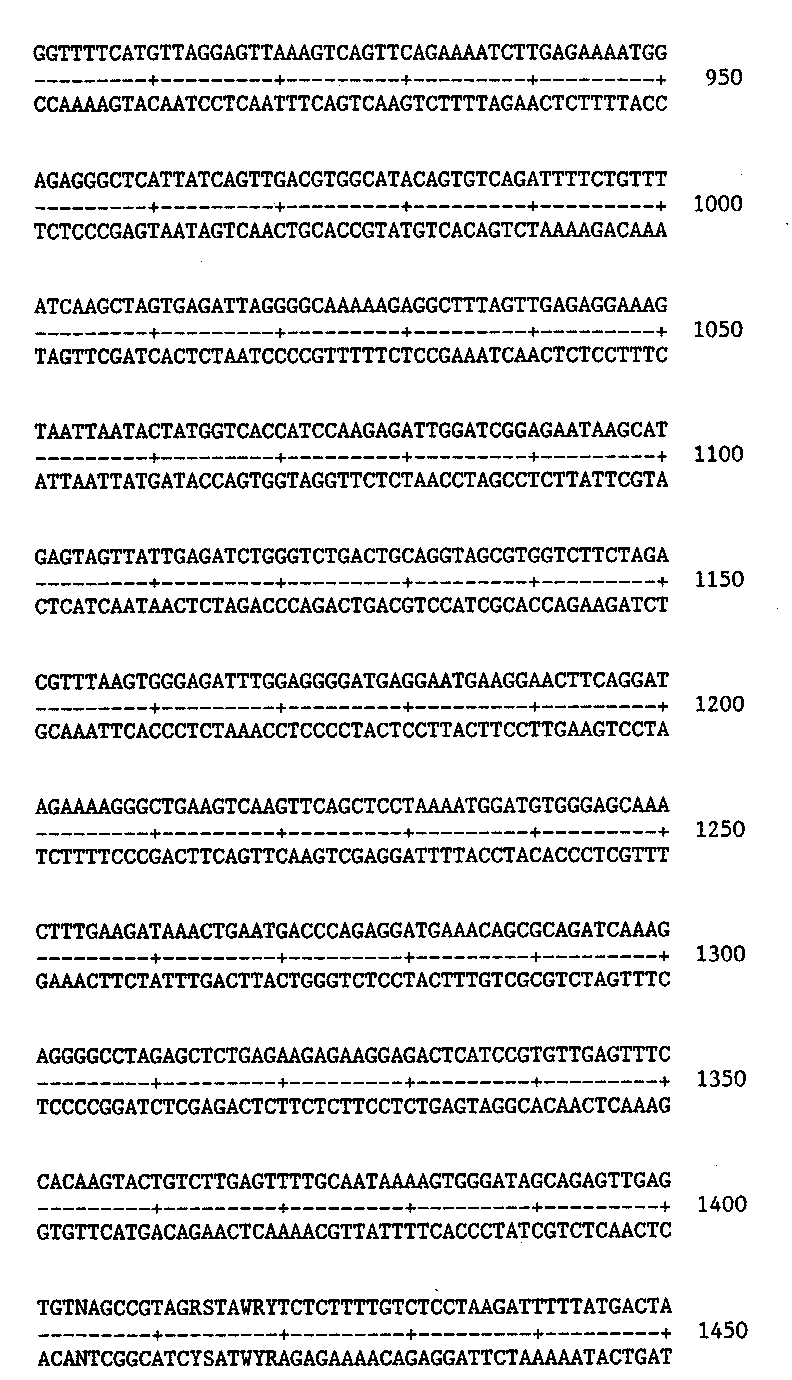

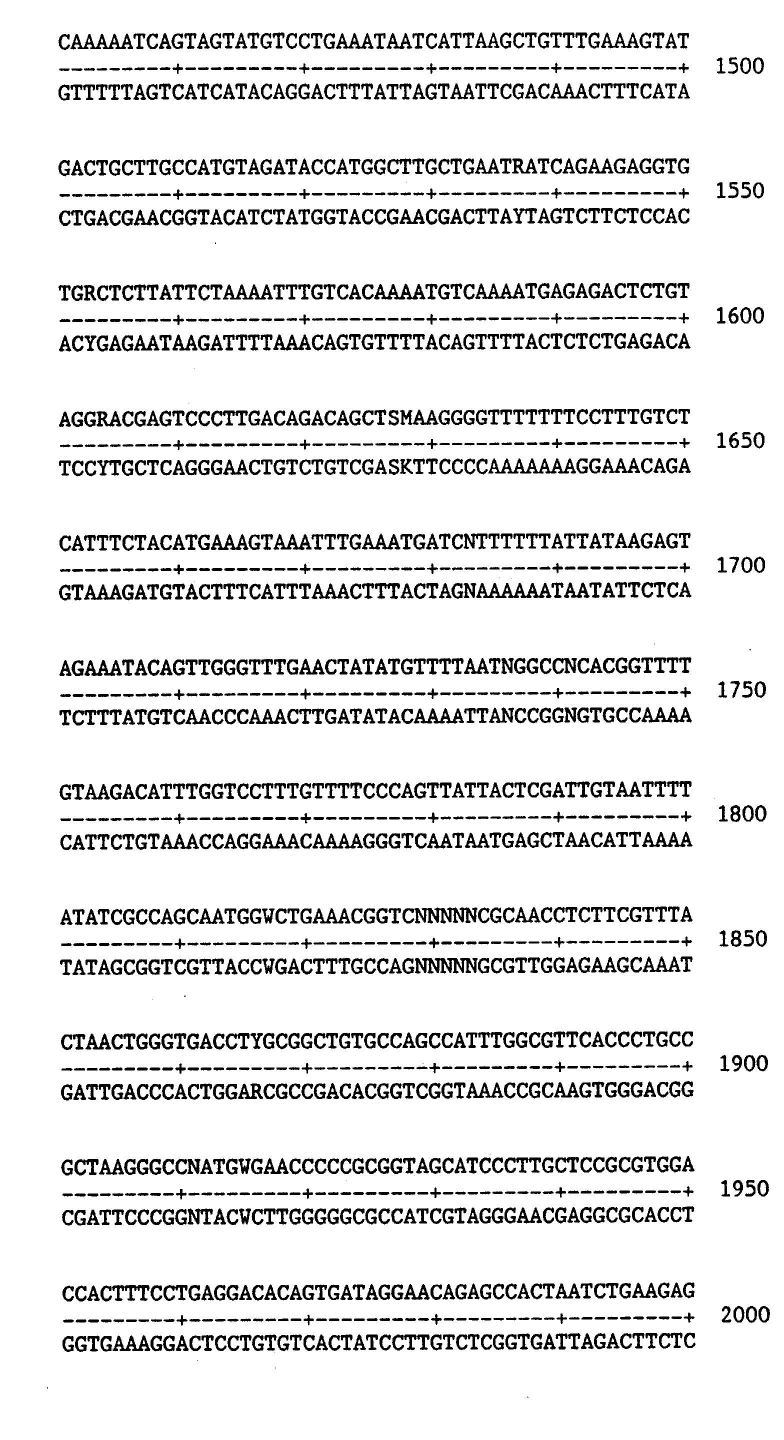

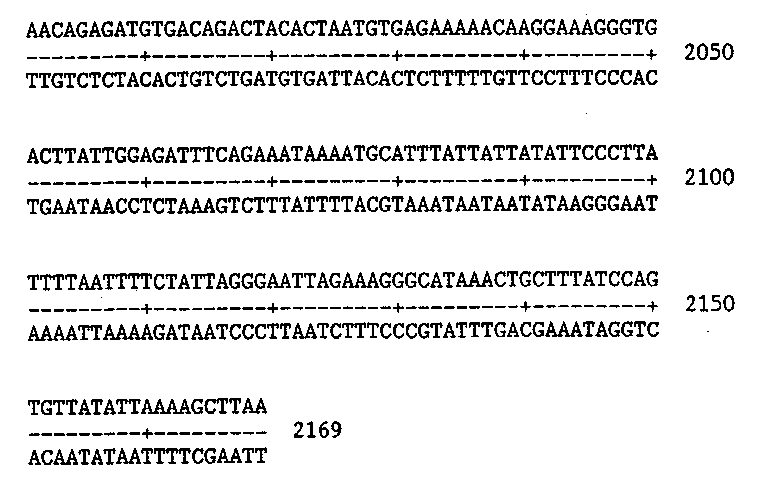

**TABLE 4**

| Reactivity Pattern of Chimeric-BMA 031 Antibodies Immunofluorescence Assay with Directly Labelled Antibodies | | | | |
|---|---|---|---|---|
| Cells Labelled with | Lymphocytes | Monocytes | Granulocytes | Erythrocytes |
| Medium | 2 ± 2^{a} | 5 ± 1 | 3 ± 2 | 1 ± 1 |
| BMA 031 | 69 ± 8 | 6 ± 3^{b} | 2 ± 3^{b} | 1 ± 1 |
| BMA 031-G1 | 69 ± 9 | 6 ± 2^{b} | 2 ± 3^{b} | 1 ± 1 |
| BMA 031-G4 | 69 ± 8 | 5 ± 3^{b} | 2 ± 3^{b} | 1 ± 1 |

| | | | | |
|---|---|---|---|---|
| ^{a}: mean values of 3 blood donors | | | | |
| ^{b}: unspecific binding by Fc-receptors was blocked | | | | |

**TABLE 5**

| T-cell Activation by Chimeric BMA 031 Antibodies Induction of γ-Interferon Release | | | | |
|---|---|---|---|---|
| T-cell activation with | | T-cell supernatant | | |
| | | d1^{a} | d2 | d3 |
| medium | | <1^{b} | <1 | <1 |
| | | | | |
| PHA | | 7 | 28 | >100 |
| | | | | |
| | (ng/ml) | | | |
| BMA 030 | 500 | 1 | 7 | 76 |
| (CD 3) | 50 | 10 | 28 | >100 |
| | 5 | 65 | 30 | >100 |
| | 0,5 | 3 | 29 | >100 |
| | | | | |
| BMA 031 | 500 | <1 | 2 | 76 |
| | 50 | <1 | 1 | 2 |
| | 5 | <1 | <1 | <1 |
| | 0,5 | <1 | <1 | <1 |
| | | | | |
| BMA 031-G1 | 500 | 2 | 5 | >100 |
| | 50 | 4 | 28 | >100 |
| | 5 | 2 | 6 | >100 |
| | 0,5 | <1 | <1 | 58 |
| | | | | |
| BMA 031-G4 | 500 | 6 | 28 | >100 |
| | 50 | 5 | 70 | >100 |
| | 5 | 3 | 28 | >100 |
| | 0,5 | <1 | <1 | <1 |

| | | | | |
|---|---|---|---|---|
| a: T-cell supernatant was harvested at day 1 after T-cell activation | | | | |
| b: γ - Interferon (ng/ml) measured by HLA-Dr induction on Colo 205-cells | | | | |

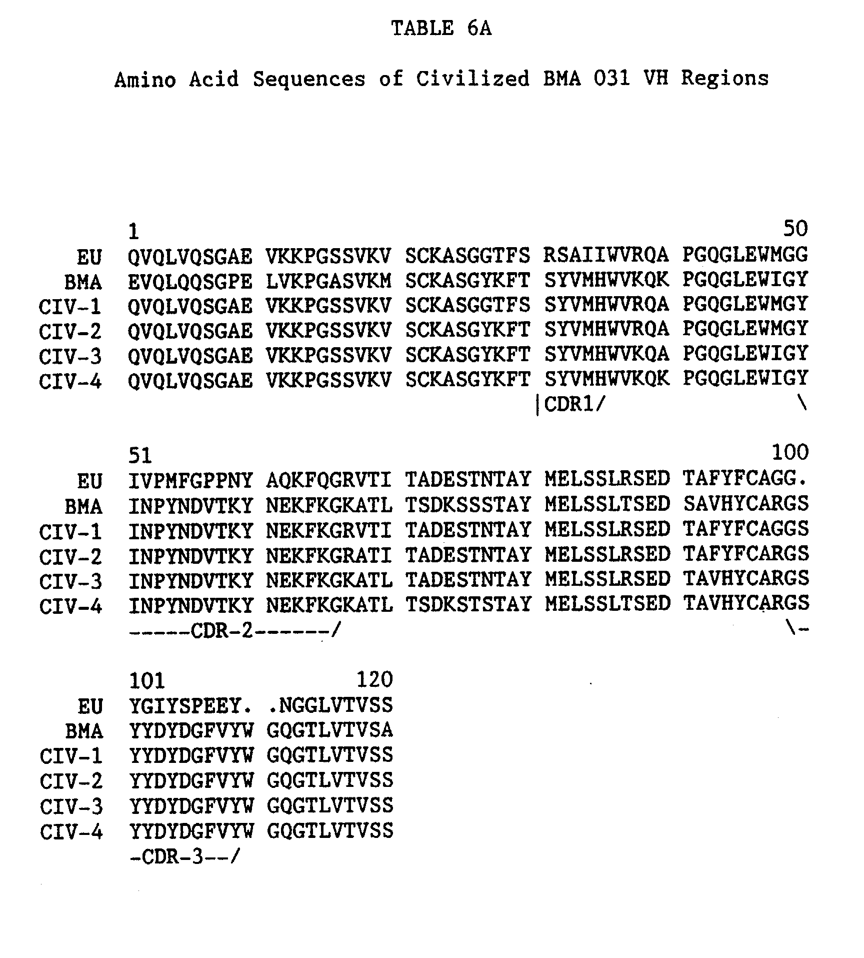

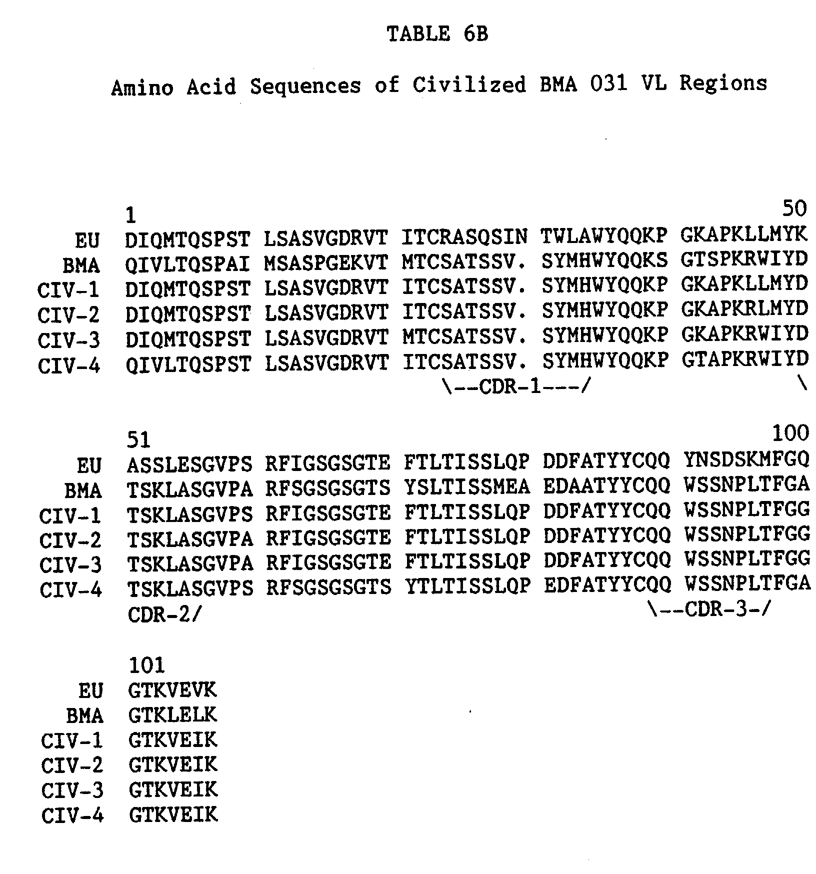

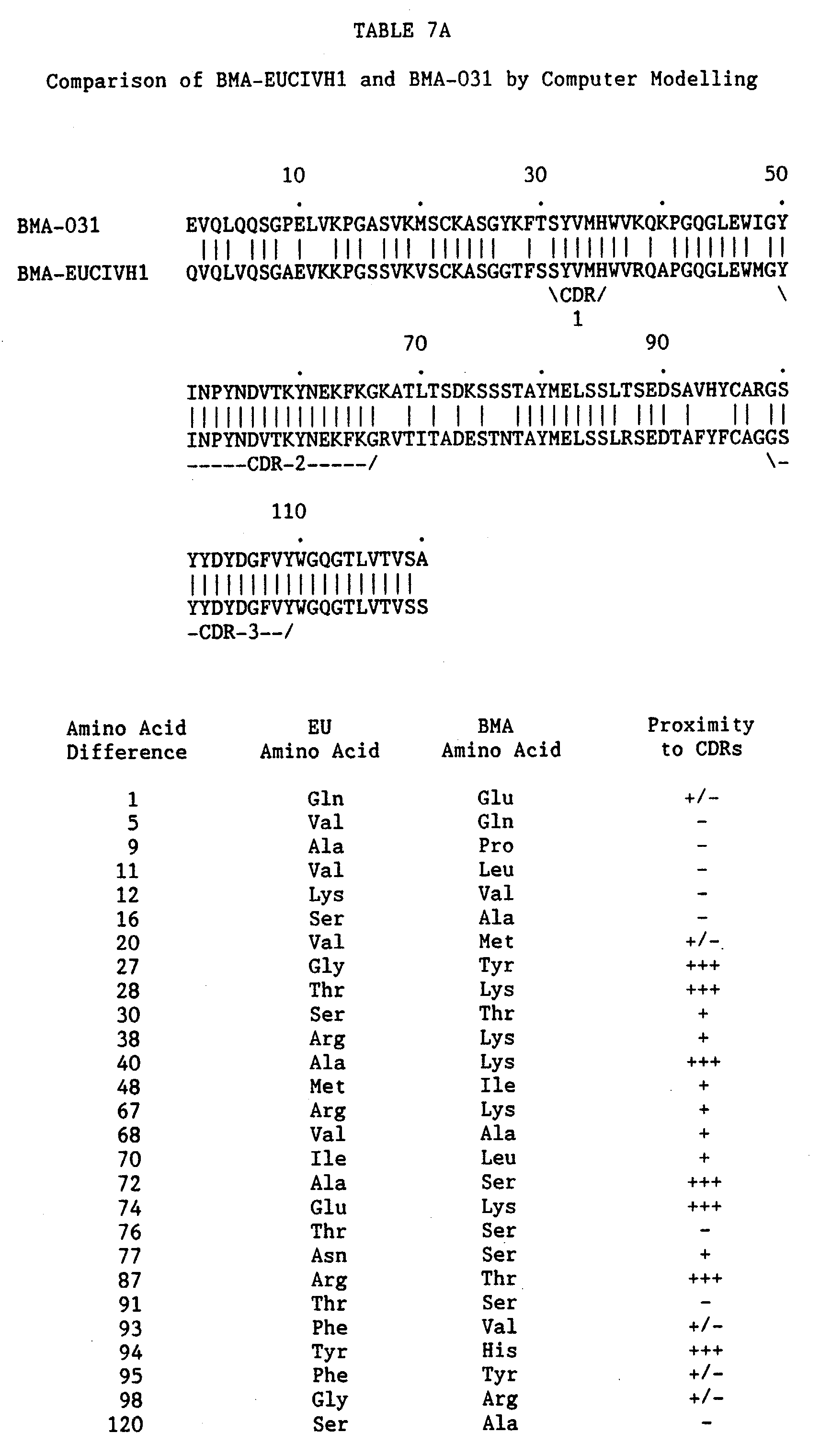

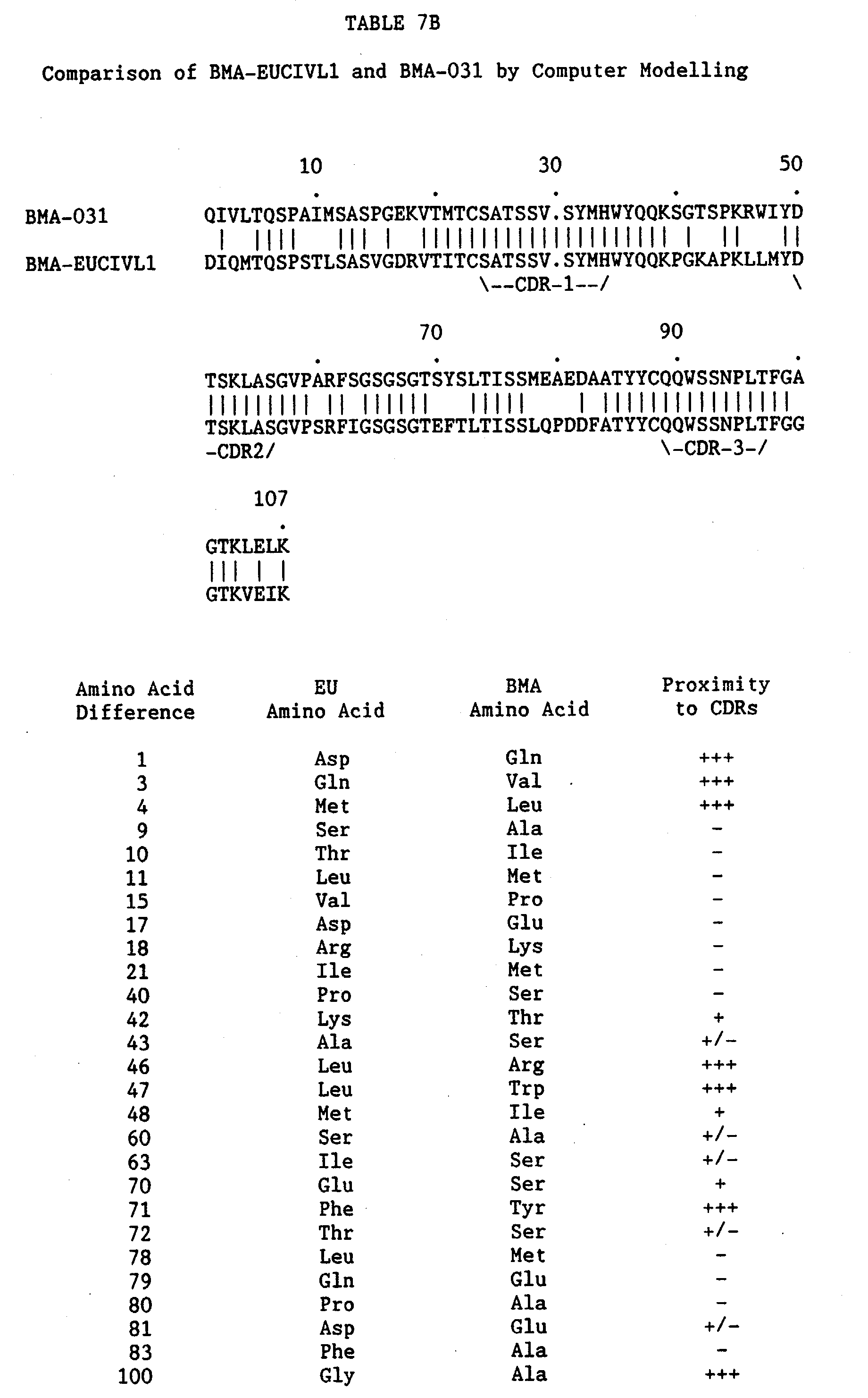

**TABLE 8**

| Amino Acid Differences Between BMA031 and EU and Their Consensus Sequences | | | | |
|---|---|---|---|---|
| Amino Acid | EU | Human | BMA031 | Mouse |
| Position | A.A. | A.A. | A.A. | A.A. |
| Heavy Chain, EU Specific: | | | | |
| 70 | Ile | + | Leu | Leu |
| 72 | Ala | + | Ser | Val |
| 74 | Glu | + | Lys | Lys |
| 93 | Phe | Val | Val | Val |
| 95 | Phe | Tyr | Tyr | Tyr |
| 98 | Gly | Arg | Arg | Arg |

| Light Chain, EU Specific: | | | | |
|---|---|---|---|---|
| 10 | Thr | Ser | Ile | Ile |
| 48 | Met | Ile | Ile | Ile |
| 63 | Ile | Ser | Ser | Ser |
| 70 | Glu | Asp | Ser | Ser |
| 81 | Asp | Glu | Glu | Glu |

| Heavy Chain, BMA Specific: | | | | |
|---|---|---|---|---|
| 1 | Gln | Gln | Gln | Gln |
| 7 | Ser | Ser | Ser | Pro |
| 9 | Ala | Ala | Pro | Ala |
| 20 | Val | Val | Met | Leu |
| 40 | Ala | Ala | Lys | Arg |
| 72 | Ala | + | Ser | Val |
| 82 | Glu | Glu | Glu | Gln |
| 94 | Tyr | Tyr | His | Tyr |

| Light Chain, BMA Specific: NONE | | | | |
|---|---|---|---|---|
| | | | | |

| | | | | |
|---|---|---|---|---|
| +, variable | | | | |

## Claims (Claims for the following Contracting State(s): AT, BE, CH, DE, DK, FR, GB, IT, LI, LU, NL, SE)

1. A chimeric antibody specific for an epitope on the human alpha/beta T-cell receptor (TCR) which is recognised by mAb BMA 031, the antibody comprising:
(a) a heavy chain whose variable region comprises the sequence of residues 1 to 120 of CIV-3 as shown in Table 6A; and
(b) a light chain whose variable region comprises the sequence of residues 1 to 107 of CIV-3 as shown in Table 6B.

2. An antibody as claimed in claim 1 wherein the heavy and light chains comprise less than the entire constant region.

3. An antibody as claimed in claim 1 or 2 which comprises a constant region derived from human antibodies.

4. An antibody as claimed in any preceding claim conjugated to a cytotoxic moiety, for example a toxin or a radioisotope.

5. An antibody as claimed in any preceding claim for use in medicine.

6. A pharmaceutical composition comprising an antibody according to any of claims 1 to 4 and a therapeutically acceptable carrier.

7. The use of an antibody as claimed in any of claims 1 to 4 in the preparation of an agent for:
(a) immunoregulation;
(b) immunosuppressive therapy;
(c) treating an autoimmune disease; and/or
(d) for mediating cytolysis, when conjugated to a cytotoxic agent.

8. An antibody as claimed in any of claims 1 to 4 which is conjugated, for example by biochemical or molecular biology techniques, to one or more isotopes and/or proteins.

9. An antibody as claimed in claim 8 wherein at least one of the proteins is an enzyme.

10. A transfectoma cell line that produces an antibody as claimed in any of claims 1 to 3.

## Claims (Claims for the following Contracting State(s): ES, GR)

1. A process for the production of a chimeric antibody specific for an epitope on the human alpha/beta T-cell receptor (TCR) which is recognised by mAb BMA 031, the antibody comprising:
(a) a heavy chain whose variable region comprises the sequence of residues 1 to 120 of CIV-3 as shown in Table 6A; and
(b) a light chain whose variable region comprises the sequence of residues 1 to 107 of CIV-3 as shown in Table 6B;
the process comprising harvesting the antibody from a cell producing the antibody.

2. A process as claimed in claim 1 wherein the heavy and light chains in the antibody comprise less than the entire constant region.

3. A process as claimed in claim 1 or 2 wherein the antibody comprises a constant region derived from human antibodies.

4. A process for producing an antibody, the process comprising conjugating an antibody produceable by a process as claimed in any preceding claim with a cytotoxic moiety.

5. A process as claimed in any of claims 1 to 4, wherein the cell is a hybridoma cell line.

6. A process for producing a pharmaceutical composition comprising an antibody produceable by a process according to any of claims 1 to 4, the process comprising admixing the antibody with a pharmaceutically acceptable carrier.

7. The use of an antibody producable by a process as claimed in any of claims 1 to 4 in the preparation of an agent for:
(a) immunoregulation;
(b) immunosuppressive therapy;
(c) treating an autoimmune disease; and/or
(d) for mediating cytolysis, when conjugated to a cytotoxic agent.

8. An antibody produceable by a process as claimed in any of claims 1 to 4 for use in medicine.

9. A process for the production of an antibody conjugate, the process comprising conjugating, for example by biochemical or molecular biology techniques, an antibody produceable by a process as claimed in any of claims 1 to 4 with one or more isotopes and/or proteins.

10. A process as claimed in claim 9 wherein at least one of the proteins is an enzyme.

11. A process for the production of a DNA construct comprising at least part of the nucleotide sequences encoding an antibody or one or more parts of an antibody produceable by a process according to any of claims 1 to 4, the process comprising coupling together successive nucleotides, and/or ligating oligo- and/or polynucleotides.

12. A transfectoma cell line that produces an antibody as described in any of claims 1 to 3.

## Patentansprüche (Patentansprüche für folgende(n) Vertragsstaat(en): AT, BE, CH, DE, DK, FR, GB, IT, LI, LU, NL, SE)

1. Ein für eine antigene Determinante auf dem menschlichen Alpha/Beta-T-Zell-Rezeptor (TCR) spezifischer Rekombinationsantikörper, der vom monoklonalen Antikörper mAb BMA 031 erkannt wird, wobei der Antikörper sich aus folgenden Bestandteilen zusammensetzt:
a) eine schwere Kette, deren variable Region eine Sequenz von CIV-3-Residuen 1 bis 120 wie in Tabelle 6A gezeigt umfaßt; und
b) eine leichte Kette, deren variable Region eine Sequenz von CIV-3-Residuen 1 bis 107 wie in Tabelle 6B gezeigt umfaßt.

2. Antikörper wie unter Patentanspruch 1, bei dem die schweren und die leichten Ketten weniger als die gesamte konstante Region umfassen.

3. Antikörper wie unter Patentanspruch 1 oder 2, der eine von menschlichen Antikörpern stammende konstante Region umfaßt.

4. Antikörper wie unter jedem beliebigen der vorhergehenden Patentansprüche, der mit einem zytotoxischen Teil konjugiert ist, beispielsweise mit einem Toxin oder einem Radioisotop.

5. Antikörper wie unter jedem beliebigen der vorhergehenden Patentansprüche zur Verwendung in der Medizin.

6. Pharmazeutische Zusammensetzung, bestehend aus einem Antikörper laut jedem beliebigen der Patentansprüche 1 bis 4 und einem vom therapeutischen Gesichtspunkt her akzeptablen Träger.

7. Benutzung eines Antikörpers laut jedem beliebigen der Patentansprüche 1 bis 4 bei der Herstellung eines Mittels für:
a) Immunregulation;
b) Immunsuppression;
c) Behandlung von Autoimmunerkrankungen; und/oder
d) zur Herbeiführung von Zellauflösung bei Konjugation mit einem zytotoxischen Mittel.

8. Antikörper wie unter jedem beliebigen der Patentansprüche 1 bis 4, der beispielsweise durch biochemische oder molekularbiologische Techniken mit einem oder mehreren Isotopen und/oder Proteinen konjugiert ist.

9. Antikörper wie unter Patentanspruch 8, bei dem mindestens eines der Proteine ein Enzym ist.

10. Transfectoma-Zellinie, die einen Antikörper wie unter jedem beliebigen der Patentansprüche 1 bis 3 beschrieben produziert.

## Patentansprüche (Patentansprüche für folgende(n) Vertragsstaat(en): ES, GB)

1. Verfahren zur Produktion eines für eine antigene Determinante auf dem menschlichen Alpha/Beta-T-Zell-Rezeptor (TCR) spezifischen Rekombinationsantikörpers, der vom monoklonalen Antikörper mAb BMA 031 erkannt wird, wobei der Antikörper sich aus folgenden Bestandteilen zusammensetzt:
a) eine schwere Kette, deren variable Region eine Sequenz von CIV-3-Residuen 1 bis 120 wie in Tabelle 6A gezeigt umfaßt; und
b) eine leichte Kette, deren variable Region eine Sequenz von CIV-3-Residuen 1 bis 107 wie in Tabelle 6B gezeigt umfaßt;
wobei das Verfahren die Ernte des Antikörpers von einer den Antikörper produzierenden Zelle umfaßt.

2. Verfahren wie unter Patentanpsruch 1, bei dem die schweren und die leichten Ketten im Antikörper weniger als die gesamte konstante Region umfassen.

3. Verfahren wie unter Patentanspruch 1 oder 2, bei dem der Antikörper eine von menschlichen Antikörpern stammende konstante Region umfaßt.

4. Verfahren zur Herstellung eines Antikörpers, wobei das Verfahren aus der Konjugation eines nach einem der unter den vorherigen Patentansprüchen angemeldeten Verfahren herstellbaren Antikörpers mit einem zytotoxischen Teil besteht.

5. Verfahren wie unter jedem beliebigen der Patentansprüche 1 bis 4, bei dem die Zelle eine Hybridomzellinie ist.

6. Verfahren zur Herstellung einer pharmazeutischen Zusammensetzung, die einen Antikörper umfaßt, der sich nach einem der unter Patentansprüchen 1 bis 4 erwähnten Verfahren herstellen läßt, wobei das Verfahren das Zumischen des Antikörpers mit einem vom pharmazeutischen Gesichtspunkt her akzeptablen Träger einschließt.

7. Benutzung eines Antikörpers, der sich nach einem der unter Ansprüche 1 bis 4 erwähnten Verfahren herstellen läßt, bei der Herstellung eines Mittels für:
a) Immunregulation;
b) Immunsuppression;
c) Behandlung von Autoimmunerkrankungen; und/oder
d) zur Herbeiführung von Zellauflösung bei Konjugation mit einem zytotoxischen Mittel.

8. Nach einem der Verfahren laut Ansprüchen 1 bis 4 herstellbarer Antikörper zur Verwendung in der Medizin.

9. Verfahren zur Herstellung eines Antikörperkonjugats, wobei das Verfahren die Konjugation - beispielsweise durch biochemische oder molekularbiologische Techniken -eines nach einem der unter Ansprüchen 1 bis 4 erwähnten Verfahren herstellbaren Antikörpers mit einem oder mehreren Isotopen und/oder Proteinen einschließt.

10. Verfahren wie unter Anspruch 9, bei dem mindestens eines der Proteine ein Enzym ist.

11. Verfahren zur Herstellung eines DNA-Gebildes, bestehend aus mindestens einem Teil der Nukleotidsequenzen, die einen Antikörper oder einen oder mehrere Teile eines Antikörpers kodieren, der sich durch eines der unter Ansprüchen 1 bis 4 erwähnten Verfahren herstellen läßt, wobei das Verfahren die Verbindung aufeinanderfolgender Nukleotide und/oder die Ligierung von Oligo- und/oder Polynukleotiden einschließt.

12. Transfectoma-Zellinie, die einen Antikörper wie unter einem der Ansprüche 1 bis 3 beschrieben produziert.

## Revendications (Revendications pour l'(les) Etat(s) contractant(s) suivant(s): AT, BE, CH, DE, DK, FR, GB, IT, LI, LU, NL, SE)

1. Un anticorps chimérique spécifique a un épitope du récepteur des cellules en T humaines, alpha/beta (RCT), reconnu par l'anticorps monoclonale (mAb) BMA 031, l'anticorps comprend:
(a) une chaîne lourde dont la région variable comprend une séquence de résidus 1 a 120 de CIV-3 comme le montre le tableau 6A; et
(b) une chaîne légère dont la région variable comprend une séquence de résidus 1 a 107de CIV-3 comme le montre le tableau 6 B.

2. Un anticorps, selon la revendication 1, où les chaînes lourdes et légères constituent moins que la totalité de la région constante.

3. Un anticorps, selon les revendications 1 ou 2, qui comprend une région constante dérivée d'anticorps humains.

4. Un anticorps, selon toutes les revendications précédentes, conjugué à une moitié cytotoxique, par exemple, une toxine ou un radio-isotope.

5. Un anticorps, selon toutes les revendications précédentes, pour utilisation en médecine.

6. Une composition pharmaceutique comprenant un anticorps conforme à chacune des revendications 1 à 4 et un porteur thérapeutique acceptable.

7. L'utilisation d'un anticorps, conforme à chacune des revendications 1 à 4, dans des préparations d'agents pour:
(a) la régulation immunitaire;
(b) la thérapie immunosuppressive;
(c) le traitement d'une maladie auto-immune; et/ou
d) pour la médiation de la cytolyse, quand il est conjugué à un agent cytotoxique

8. Un anticorps, conforme à chacune des revendications 1 à 4, qui est conjugué, par exemple par des techniques biochimiques ou de biologie moléculaire, à un ou plusieurs isotopes et/ou protéines.

9. Un anticorps, selon la revendication 8, où au moins une des protéines est une enzyme.

10. Une lignée de cellules de transfectomes qui produit un anticorps conforme à chacune des revendications de 1 à 3.

## Revendications (Revendications pour l'(les) Etat(s) contractant(s) suivant(s): ES, GR)

1. Un procédé pour la production d'un anticorps chimérique spécifique à un isotope des récepteurs des cellules en T humaines alpha beta (RCT) reconnu par l'anticorps monoclonale (mAb) BMA 031, cet anticorps comprenant:
a) une chaîne lourde dont la région variable comprend une séquence de résidus 1 a 120 deCIV-3 comme le montre le tableau 6 A; et
b) une chaîne légère dont la région variable comprend une séquence de résidus 1 a 107de CIV-3 comme le montre le tableau 6 B;
le procédé consiste a récolter des anticorps d'une cellule qui produit l'anticorps.

2. Un procédé, selon la revendication 1, par lequel dans l'anticorps, les chaînes lourdes et légères constituent moins que la totalité de la région constante.

3. Un procédé, selon les revendications 1 et 2, par lequel les anticorps comprennent une région constante dérivée d'anticorps humains.

4. Un procédé pour produire un anticorps, le procédé consistant à conjuguer un anticorps, que l'on peut produire par le procédé conforme à toutes les revendications précédentes, avec une moitié cytotoxique.

5. Un procédé, conforme à chacune des revendications de 1 à 4, où la cellule est une lignée de cellules d'hybrydomes.

6. Un procédé pour produire une composition pharmaceutique consistant d'un anticorps qui peut être produit par un procédé conforme à chacune des revendications 1 à 4, ce procédé comprenant l'ajout et le mélange de cet anticorps à un porteur pharmaceutique acceptable.

7. L'utilisation d'un anticorps, produit par un procédé conforme à chacune des revendications 1 à 4, dans la préparation d'un agent pour:
(a) la régulation immunitaire
(b) la thérapie immunosuppressive;
(c) le traitement d'une maladie auto-immune; et/ou
(d) pour la médiation de la cytolyse, quand il est conjugue à un agent cytotoxique

8. Un anticorps pour utilisation en médecine, qui peut être produit par un procédé conforme à chacune des revendications de 1 à 4.

9. Un procédé pour la production d'un conjugué d'anticorps, le procédé consistant à conjuguer, par exemple par des techniques biochimiques ou de biologie moléculaire, un anticorps, qui peut être produit par un procédé à chacune des revendications 1 à 4, à un ou plusieurs isotopes et/ou protéines.

10. Un procédé, selon dans la revendication 9, où au moins une des protéines est une enzyme.

11. Un procédé pour la production d'une structure de l'ADN comportant au moins une portion de séquences nucléotides qui encodent un anticorps ou une ou plusieurs partie d'un anticorps produit par un procédé conforme à toutes les revendications de 1 à 4, le procédé consistant en l'accouplement de nucléotides successifs, et/ou en la ligature d'oligo et/ou de poly nucléotides.

12. Une lignée de cellules de transfectomes qui produit un anticorps conforme à chacune des revendications 1 à 3.
